# EUROPÄISCHE PATENTSCHRIFT

(11) **EP 1 257 819 B1**
(45) Veröffentlichungstag und Bekanntmachung des Hinweises auf die Patenterteilung: **06.08.2008**
(21) Anmeldenummer: 01951152.6
(22) Anmeldetag: 24.01.2001
(51) Int. Cl.: G01N 33/52, G01N 33/68

(54) **VERFAHREN ZUR IDENTIFIZIERUNG VON SUBSTANZEN, DIE DIE AKTIVITÄT VON HYPERPOLARISATIONS-AKTIVIERTEN KATIONEN-KANÄLEN MODULIEREN**
METHOD FOR IDENTIFYING SUBSTANCES WHICH MODULATE THE ACTIVITY OF HYPERPOLARISATION-ACTIVATED CATION CHANNELS
PROCEDE D'IDENTIFICATION DE SUBSTANCES QUI MODULENT L'ACTIVITE DE CANAUX DE CATIONIQUES ACTIVES PAR HYPERPOLARISATION

(30) Priorität: 12.02.2000 DE 10006309
(43) Veröffentlichungstag der Anmeldung: 20.11.2002
(73) Patentinhaber: Sanofi-Aventis Deutschland GmbH, 65929 Frankfurt am Main (DE)
(72) Erfinder: JANSEN, Hans-Willi, 65527 Niedernhausen (DE); BRÜGGEMANN, Andrea, CH - 2084 Carrouge (VD) (CH); HEITSCH, Holger, 55252 Mainz-Kastel (DE); GÖGELEIN, Heinz, 60528 Frankfurt am Main (DE)
(86) Internationale Anmeldenummer: PCT/EP2001/000755
(87) Internationale Veröffentlichungsnummer: WO 2001/059153

(56) Entgegenhaltungen:
- WO-A-00/63349
- WO-A-00/73431
- WO-A-99/11784
- LANGHEINRICH U ET AL: "HYPERPOLARIZATION OF ISOLATED CAPILLARIES FROM GUINEA-PIG HEART INDUCED BY K+ CHANNEL OPENERS AND GLUCOSE DEPRIVATION" JOURNAL OF PHYSIOLOGY, XX, XX, Bd. 502, Nr. 2, Juli 1997 (1997-07), Seiten 397-408, XP001058204 ISSN: 0022-3751

## Beschreibung

Gegenstand der vorliegenden Erfindung ist ein Verfahren zur Identifizierung von Substanzen, welche die Aktivität von hyperpolarisations-aktivierten Kationen-Kanälen modulieren, und die Verwendung des Verfahrens.

Einige Gene muriner und humaner hyperpolarisations-aktivierter Kationen-Kanäle sind bereits bekannt, beispielsweise muHCN2(muHAC1) (Ludwig et al. (1998)), huHCN4 (Ludwig et al. (1999)), huHCN2 (Vaccari,T. et al. (1999) Biochim. Biophys. Acta 1446(3): 419-425), WO 99/32615 und WO 99/42574.

In Ludwig et al. (1998) wurde gezeigt, daß sich muHCN2 in HEK293-Zellen transient transfizieren läßt und daß sich der entsprechende Kanal elektrophysiologisch (Patch-Clamp Untersuchungen) in den transfizierten Zellen gut untersuchen läßt. In seinen elektrophysiologischen Eigenschaften entspricht der klonierte Kanal dem in Schrittmacherzellen beschriebenen I_{f}- oder Iₕ-Strom, der bisher auf molekularer Ebene nicht bekannt war (Ludwig et al. (1998), Biel et al. (1999)). Der Kanal aktiviert, wenn man das Haltepotential in Richtung Hyperpolarisation (Potential auf ca. -100 bis -160 mV) verändert. Die Patch-Clamp Technik läßt sich jedoch nicht automatisieren und ist nicht für ein Hochdurchsatz-Screening (HTS) geeignet.

Im FLIPR (Fluorescence Imaging Plate Reader; Molecular Devices, Sunnyvale, CA, USA) können mit geeigneten Farbstoffen Ionenströme gemessen werden. Ein- oder Ausstrom von Ionen führt zu Änderungen des Membranpotentials, die man im FLIPR mit geeigneten Fluoreszenzfarbstoffen im Hochdurchsatz-Screening messen kann. Im FLIPR lassen sich jedoch keine Spannungssprünge erzeugen, wie es beim Patch-Clamp Verfahren möglich ist. Spannungssprünge sind aber für die Aktivierung von hyperpolarisations-aktivierten Kationen-Kanälen eine notwendige Voraussetzung. Um möglichst viele Substanzen untersuchen zu können, wurde ein Verfahren entwickelt, das ein Hochdurchsatz-Screening für Modulatoren eines hyperpolarisations-aktivierten Kationen-Kanals ermöglicht.

In diesem Zusammenhang war es eine Aufgabe der vorliegenden Erfindung, einen Weg zu finden, die Zellen, die den hyperpolarisations-aktivierten Kationen-Kanal exprimieren, zu hyperpolarisieren (d.h. den hyperpolarisations-aktivierten Kationen-Kanal zu aktivieren) und diese Hyperpolarisation der Zelle aufrechtzuerhalten. Unter physiologischen Bedingungen wird eine Hyperpolarisation der Zelle, die ausreicht, einen hyperpolarisations-aktivierten Kationen-Kanal zu aktivieren, durch die Aktivität dieses Kanals wieder aufgehoben. Nur wenn die Hyperpolarisation aufrechterhalten wird, kann - beispielsweise im FLIPR - die Depolarisation der Zelle, die unter geeigneten Bedingungen durch eine die Aktivität des hyperpolarisations-aktivierten Kationen-Kanals modulierende Substanz hervorgerufen wird, gemessen werden.

Gegenstand der vorliegenden Erfindung ist ein Verfahren zur Untersuchung von hyperpolarisations-aktivierten Kationen-Kanälen, wobei Zellen, die den hyperpolarisations-aktivierten Kationen-Kanal exprimieren, hyperpolarisiert werden - d.h. der hyperpolarisations-aktivierte Kationen-Kanal aktiviert wird - und diese Hyperpolarisation der Zellen, die unter physiologischen Bedingungen durch die Aktivität des hyperpolarisations-aktivierten Kationen-Kanals wieder aufgehoben wird, aufrechterhalten wird. Durch Zugabe von extrazellulären Natriumionen wird dem aktivierten HCN-Kanal im FLIPR die Möglichkeit gegeben, Natriumionen in die Zellen zu transportieren, die Zellen also zu depolarisieren. Werden gleichzeitig mit oder bereits vor der Zugabe der Natriumionen Substanzen gegeben, die die Aktivität des hyperpolarisations-aktivierten Kationen-Kanals modulieren, so wird die Depolarisation gegenüber alleiniger Natriumionen-Gabe bei HCN-Aktivatoren (z.B. Forskolin) verstärkt und bei HCN-Inhibitoren (z.B. Zatebradine = 3-[3-[[2-(3,4-Dimethoxyphenyl)-ethyl]-methylamino]-propyl]-1,3,4,5-tetrahydro-7,8-dimethoxy-2H-3-benzazepin-2-on aus Reiffen et al. (1990)) abgeschwächt.

Durch die Messung der Depolarisation der Zellen bzw. der Veränderung ihres Membranpotentials, können Substanzen identifiziert werden, die die Aktivität des hyperpolarisations-aktivierten Kationen-Kanals modulieren.
Gegenstand der Erfindung ist ein Verfahren zur Identifizierung von Substanzen, die die Aktivität von hyperpolarisations-aktivierten Kationen-Kanälen modulieren, wobei
a) Zellen, die einen hyperpolarisations-aktivierten Kationen-Kanal exprimieren, verwendet werden;
b) die Zellen in Anwesenheit eines potentialsensitiven Fluoreszenzfarbstoffes mit einem isoosmolaren, Natriumionen-freien Puffer hyperpolarisiert werden; und
c) die Veränderung des Membranpotentials der Zellen nach gleichzeitiger Gabe von Natriumionen und der zu untersuchenden Substanz registriert wird.

Gegenstand der Erfindung ist ein Verfahren zur Identifizierung von Substanzen, welche die Aktivität von hyperpolarisations-aktivierten Kationen-Kanälen modulieren, wobei
a) Zellen, die einen hyperpolarisations-aktivierten Kationen-Kanal exprimieren, verwendet werden;
b) die Zellen in Anwesenheit eines potentialsensitiven Fluoreszenzfarbstoffes mit einem isoosmolaren, Natriumionen-freien Puffer hyperpolarisiert werden;
c) die Zellen mit einer zu untersuchenden Substanz inkubiert werden; und
d) die Veränderung des Membranpotentials der Zellen nach Zugabe von Natriumionen registriert wird.

Für die Funktion von hyperpolarisations-aktivierten Kationen-Kanälen, z. B. der HCN (HAC)-Kanäle, ist die Anwesenheit extrazellulärer Kaliumionen wichtig, d.h. der isoosmolare, Natriumionen-freie Puffer enthält Kaliumionen (K⁺) - z. B. in From von Kaliumchlorid - vorzugsweise mindestens ca. 0,5 mM K⁺ bzw. 0,8 mM K⁺, vorzugsweise 2 mM, besonders bevorzugt ca. 5 mM K⁺.

Vorzugsweise enthält der isoosmolare, Natriumionen-freie Puffer ein anderes Kation, das nicht membrangängig ist, in Mengen, die der normalen extrazellulären Natriumionen-Konzentration entsprechen, z.B. Cholin⁺ beispielsweise in Form von Cholinchlorid oder NMDG (N-Methyl-D-glucamin).

Vorzugsweise enthält der isoosmolare, Natriumionen-freie Puffer einen potentialsensitiven Farbstoff, beispielsweise einen potentialsensitiven Fluoreszenzfarbstoff, z.B. ein Oxonolderivat wie 3-Bis-barbitursäure-oxonol. Vorzugsweise werden potentialsensitive Fluoreszenzfarbstoffe verwendet, die für die Untersuchung des Membranpotentials nichterregbarer Zellen geeignet sind, beispielsweise potentialsensitive slow-response Farbstoffe, z.B. Bis-(1,3-dibutylbarbitursäure)-trimethin oxonol [DiBAC₄(3)], Bis-(1,3-diethylthiobarbitursäure)-trimethin oxonol [DiSBAC₂(3)] oder Bis-(1,3-dibutylbarbitursäure)-pentamethin oxonol [DiBAC₄(5)]. Weitere bekannte und geeignete potentialsensitive Farbstoffe sind schnelle Farbstoffe (zumeist vom Styrylpyridinium-Typ), die vorzugsweise für erregbare Zellen wie Neuronen, kardiale Zellen usw. verwendet werden können. Diese potentialsensitiven Farbstoffe reagieren im Millisekundenbereich und sind nicht sehr sensitiv (2-10% Fluoreszenzänderung bei 100 mV Potentialänderung). Andererseits können langsame Farbstoffe, z.B. vom Carbocyanin-Typ verwendet werden, z.B. DiOC5(3) 3,3'dipentyloxacarbo-cyanine iodide, DiOC6(3) 3,3'-dihexyloxacarbocyanine iodide usw.), JC-1 (5,5',6,6'-tetrachloro-1,1',3,3'-tetraethylbenzimidazolcarbocyanine iodide) sowie Rhodamine 123; diese langsamen potentialsensitiven Farbstoffe sind vor allem für Untersuchungen des Membranpotentials von Mitochondrien geeignet.

Eine besondere Ausführungsform der Erfindung betrifft die Verwendung des Fluoreszenzfarbstoffs aus dem "FLIPR Membrane Potential Assay Kit" (Molecular Devices Sunnyvale, CA, USA). Die Fluoreszenz dieses Farbstoffs lässt sich entweder mit dem Standard Emmissionsfilter messen, der zwischen 510 und 580 nm durchlässig ist, bevorzugt aber mit einem Filter, der oberhalb von 550 nm durchlässig ist. Welcher Farbstoff, oder welche Kombination von Farbstoffen diesem Kit zugrundeliegen, ist nicht bekannt. Die Firma gibt für ihren Kit mehrere Vorteile gegenüber DiBAC₄(3) an:
1) Die Durchführung von Membranpotentialmessungen mit dem Kit ist nicht temperatursensitiv, ganz im Gegenteil zu DiBAC₄(3), bei dem vor Beginn der eigentlichen Messung im FLIPR eine Temperatur-Äquilibrierung stattfinden muss.
2) Das im FLIPR zugegebene Volumen kann größer sein als bei DiBAC₄(3), bei dem üblicherweise alle Substanzen 10fach konzentriert zugegeben werden müssen.
3) Messungen sind viel schneller durchführbar, da der Kit eine viel kürzere Zeit bis zur Gleichgewichtseinstellung braucht als DiBAC₄(3), das üblicherweise zwischen 10-30 Minuten benötigte.
4) Für viele Messprotokolle ist vor Farbstoffzugabe kein Waschschritt mehr nötig.
5) Der Farbstoff muss nicht in jeder Lösung vorhanden sein.

Von diesen Vorteilen sind besonders die ersten beiden für die hyperpolarisations-aktivierten Kationen-Kanäle von Bedeutung, da besonders im FLIPR 11, der die Messung in 384-Well-Platten ermöglicht und bevorzugt für das Hochdurchsatz-Screening eingesetzt wird, die Temperierung nur sehr umständlich und zeitaufwendig durchführbar ist. Bei schwerlöslichen Substanzen ist es außerdem von großem Vorteil, wenn sie statt 10fach nur fünf-, drei- oder gar zweifach konzentriert zu den Zellen gegeben werden müssen.

Vorzugsweise werden in dem Verfahren Zellen verwendet, deren intrazelluläre cAMP Konzentration erhöht ist. Das kann beispielsweise durch Zugabe von membrangängigen cAMP-Derivaten erreicht werden, z.B. Dibutyryl-cAMP und 8-Bromo-cAMP. Eine weitere Möglichkeit, die intrazelluläre cAMP Konzentration zu erhöhen, ist die Zugabe eines Aktivators der Adenylatzyklase, z.B. Forskolin, vorzugsweise in Konzentrationen von 1 µM-100 µM oder weniger als 1 µM Forskolin, besonders bevorzugt etwa 10µM Forskolin. Prinzipiell können auch alle Substanzen bzw. Liganden verwendet werden, die über Signaltransduktion in der verwendeten Zellinie die Adenylatzyklase aktivieren (z.B. Liganden für β-adrenerge Rezeptoren wie z.B. Adrenalin, Isoproterenol, Noradrenalin usw., falls die Zelle endogene β-adrenerge Rezeptoren hat).
Zur Depolarisation des Membranpotentials wird im FLIPR Na⁺ (zumeist in Form von NaCl) zu den durch den Natriumionen-freien Puffer hyperpolarisierten Zellen gegeben, vorzugsweise auf eine Na⁺-Endkonzentration von 20-100 mM, besonders bevorzugt auf eine Na⁺-Endkonzentration von 50 mM. Bei Verwendung des FLIPR Membrane Potential Assay Kits (Molecular Devices, Sunnyvale, CA, USA), kann die Na⁺-Endkonzentration ebenfalls zwischen 20-100 mM liegen, bevorzugt zwischen 40 bis 80 mM.

Insbesondere betrifft die Erfindung Verfahren, in denen der hyperpolarisationsaktivierbare Kationen-Kanal ein HCN1-, HCN2-, HCN3-, HCN4-Kanal (wobei HAC1=HCN2, HAC2=HCN1, HAC3=HCN3 und HAC4=HCN4 ist) oder ein KAT1 (=AKT) Kanal (hyperpolarisations-aktivierter Kalium-Kanal aus Arabidopsis thaliana) oder ein Heteromultimeres dieser Kanäle (d. h. ein Kanal, der aus Untereinheiten verschiedener hyperpolarisations-aktivierter Kationen-Kanäle zusammengesetzt ist) oder ein chimärer hyperpolarisations-aktivierter Kationen-Kanal (d. h. ein synthetischer Kanal, bei dem einzelne Untereinheiten aus Teilen verschiedener Kanäle bzw. hyperpolarisations-aktivierter Kationen-Kanäle zusammengesetzt sind). Vorzugsweise ist der hyperpolarisations-aktivierte Kationen-Kanal ein humaner hyperpolarisations-aktivierter Kationen-Kanal, z.B. huHCN2 (SEQ ID NO. 1, SEQ ID NO. 2) oder huHCN4 (SEQ ID NO. 3, SEQ ID. NO.4) oder ein muriner hyperpolarisations-aktivierter Kationen-Kanal muHCN2 (SEQ ID NO. 5, SEQ ID NO. 6). Auf Aminosäureebene beträgt die Identität zwischen muHCN2 und huHCN2
94,8 %.
Das Verfahren eignet sich prinzipiell für alle Kationenkanäle, die durch Hyperpolarisation aktiviert werden, d.h. z.B. für HCN1-4 (bzw. HAC1-4; s. Biel et al. 1999).

Die Zellen sind vorzugsweise Säugerzellen, beispielsweise CHO oder HEK293 Zellen. Vorzugsweise wird CHO oder eine andere Zellinie mit vergleichbar geringen endogenen Kalium-Kanälen verwendet, da diese endogene Kalium-Kanäle die Messung im FLIPR stören können. Es können aber auch Zellen verwendet werden, deren endogenen Kalium-Kanäle nicht funktionell exprimiert werden (z.B. entsprechende knock-out Zellen).

Vorzugsweise enthalten die Zellen eine DNA, die für den hyperpolarisations-aktivierten Kationen-Kanal kodiert, vorzugsweise die cDNA eines hyperpolarisations-aktivierten Kationen-Kanals in einem geeigneten Plasmid. Vorzugsweise werden solche Zellen dadurch hergestellt, daß die Ausgangszellinie mit einem Plasmid, das die cDNA eines hyperpolarisations-aktivierten Kationen-Kanals enthält, transfiziert wird.

Im Falle der hyperpolarisations-aktivierten Kationen Kanäle sollen Änderungen im Membranpotential der Zellen registriert werden, die aus der Aktivierung oder aus der Inhibition dieser Kanäle resultieren. Die drei Bis-barbitursäure-oxonole (Information aus: Handbook of Fluorescent Probes and Research Chemicals, 6^{th} edition, Molecular Probes, Eugene OR,USA), die meistens als DiBAC-Farbstoffe bezeichnet werden, bilden eine Familie potentialsensitiver Farbstoffe mit Exzitationsmaxima bei 490 nm (DiBAC₄(3)), 530 nm (DiSBAC₂(3)) und 590 nm (DiBAC₄(5)). Die Farbstoffe gelangen in depolarisierte Zellen, in denen sie intrazelluläre Proteine oder Membranen binden, was zu verstärkter Fluoreszenz und zu Rotverschiebung führt. Hyperpolarisation führt zu einem Ausstoßen der anionischen Farbstoffe und somit zu einer Fluoreszenzabnahme. Diese Fluoreszenzabnahme kann gemessen werden, z.B. mit dem Meßgerät FLIPR.

Der FLIPR (für: Fluorescent Imaging Plate Reader; Hersteller: Molecular Devices, Sunnyvale CA, USA) ist ein Meßgerät, mit dem man Veränderungen der Fluoreszenzintensität in allen Wells einer Mikrotiterplatte gleichzeitig messen kann. Die Anregung der eingesetzten Farbstoffe geschieht mittels eines in das System integrierten Argon-Lasers bei 488 nm. Der Standard-Emissionsfilter des Systems ist durchlässig im Bereich von 510 - 580 nm. Die emittierte Fluoreszenz wird mit einer CCD-Kamera registriert, wobei das System es erlaubt, die Fluoreszenz in allen Wells einer 96-Well- oder 384-Well-Mikrotiterplatte in einem Minimalabstand von ca. einer Sekunde gleichzeitig zu registrieren. Durch einen eingebauten Pipettor kann auch während der Substanzzugabe die Fluoreszenz bestimmt werden, was besonders bei schnellen Prozessen wichtig ist. Mittels einer besonderen Optik wird erreicht, daß nur die Fluoreszenz in einer Schicht von ca. 50 µm registriert wird, nicht aber in dem ganzen Well. Dies ist zur Hintergrundsverringerung für alle Messungen wichtig, bei denen der Fluoreszenzfarbstoff auch extrazellulär vorliegt, wie z.B. auch bei Messung von Membranpotentialänderungen mit den DiBAC-Farbstoffen. Standardanwendungen des Systems sind Messungen der intrazellulären Calcium-Konzentration bzw. des Membranpotentials von Zellen.
Von den oben genannten Farbstoffen weist DiBAC₄(3), das sich aufgrund seines Exzitationsmaximums am besten für den Argon-Laser im FLIPR eignet, die höchste Sensitivität für Spannungsunterschiede auf.
Da die DiBAC₄(3)-Fluoreszenz empfindlich auf Temperaturschwankungen reagiert kann, wird vorzugsweise nach einer bestimmten Inkubationszeit bei 37°C, z.B. nach 30 Minuten, möglichst schnell gemessen, da sonst durch eine Abkühlung der Färbelösung das Meßergebnis verfälscht werden kann. Vorzugsweise wird vor Beginn der Messung eine fünfminütige Präinkubationsphase durchgeführt, um Temperaturschwankungen auf der Mikrotiterplatte auszugleichen. Die Messung wird mit den vom FLIPR-Hersteller für Membranpotentialmessung voreingestellten Temperatur-Parametem (ca. 37°C) durchgeführt.
Vorzugsweise wird das Volumen der Reaktionslösung, in der das Verfahren durchgeführt wird, im FLIPR möglichst wenig verändert, da die Reproduzierbarkeit des DiBAC₄(3)-Signals am besten ist, wenn im FLIPR nur eine relativ geringe Volumenänderung vorgenommen wird. Vorzugsweise werden die zu testenden Substanzen daher 10-fach konzentriert zu den DiBAC₄(3)-gefärbten Zellen gegeben.

Da die Fluoreszenzmessung mit dem "FLIPR Membrane Potential Assay Kit" nicht temperatursensitiv ist, lässt sie sich einfach bei Raumtemperatur durchführen, was besonders für den FLIPR II, mit dem Messungen mit 384-Well-Mikrotiterplatten möglich sind, vorteilhaft ist.
Ein weiterer Vorteil des "FLIPR Membrane Potential Assay Kit " gegenüber DiBAC₄(3) ist, daß man die Substanzen nicht 10fach konzentriert zugeben muss.

Die HCN-Kanäle werden durch Hyperpolarisation aktiviert (z.B. HCN2 bei -100 mV zu 50%) und bewirken eine Depolarisation der Zellen. Durch Erhöhung der intrazellulären cAMP-Konzentration (z.B. über Dibutyryl-cAMP oder über Forskolin) läßt sich der Wert der halbmaximalen Aktivierung um ca. 10 mV zu positiveren Potentialen verschieben (Ludwig et al., 1998).
Elektrophysiologisch lassen sich HCN-Kanäle an stabil transfizierten Zellen mittels der Patch-Clamp Methode gut untersuchen, da Spannungssprünge leicht durchführbar sind.
Im FLIPR dagegen sind keine Spannungssprünge durchführbar, und eine Hyperpolarisation der Zellen wäre gerade aufgrund der HCN-Aktivität nur transient. Es ist nicht gelungen, durch die Zugabe eines HCN2-Hemmers (Zatebradine) eine Hyperpolarisation der transfizierten Zellen zu erreichen, weil das Ruhemembranpotential der transfizierten Zellen viel zu weit von den Potentialen entfernt liegt, bei denen HCN2 aktiviert wird.
Einerseits ist zur HCN-Aktivierung eine Hyperpolarisation nötig, andererseits führt ein aktivierter HCN unter physiologischen Bedingungen umgehend zur Depolarisation. In der vorliegenden Erfindung wurden deshalb Bedingungen gewählt, die HCN-Kanäle durch Hyperpolarisation aktivieren können, die Depolarisation durch den aktivierten HCN-Kanal jedoch zunächst unmöglich machen. Die auf Mikrotiterplatten ausgesäten Zellen werden dafür in einem isoosmolaren Puffer gewaschen, bei dem NaCl durch Cholinchlorid ersetzt ist. Dieser Waschpuffer enthält aber noch 5 mM KCI, da extrazelluläres K⁺ für die HCN-Aktivität von Bedeutung ist (Biel et al. 1999). Dieser Waschpuffer, der zur Hyperpolarisation der HCN-Zellen dient, enthält gleichzeitig 5 µM DIBAC₄(3) zur Messung von Membranpotentialänderungen im FLIPR. Durch das Entfernen des extrazellulären Na⁺ werden die Zellen hyperpolarisiert, der HCN also aktiviert. Er kann aber nicht zu einer Depolarisation der Zellen führen, da entsprechende Konzentrationsgradienten der von HCN transportierten Ionen Na⁺ oder K⁺ fehlen. Ein aktivierter HCN könnte jetzt nur zu einer stärkeren Hyperpolarisation führen. Dies spiegelt sich darin wieder, daß bei HCN-Zellen die im FLIPR gemessene Ausgangsfluoreszenz bei 10 µM Forskolin niedriger liegt als ohne Forskolin, während es bei untransfizierten Zellen keinen Unterschied gibt.
Im FLIPR wird Na⁺ zu den Zellen gegeben, so daß der aktivierte HCN (nach einigen Sekunden, in denen es zu Mischungseffekten kommt) ab etwa 15 Sekunden nach Na⁺-Zugabe zu einer Depolarisation der Zellen führt, die durch Zunahme der Fluoreszenz sichtbar wird. Entscheidend für das Auffinden von HCN-Modulatoren ist ein deutlicher Unterschied zwischen Zellen mit aktivem HCN-Kanal (nur Na⁺-Zugabe) und Zellen mit blockiertem HCN-Kanal (Na⁺ + 8mM CsCl). Eine Aktivierung des HCN-Kanals durch Vorinkubation mit 10 µM Forskolin vergrößert den Abstand des ungehemmten 100%-Wertes vom gehemmten 0%-Wert deutlich (s. Abb. 1).

Eine besondere Ausführungsform der Erfindung betrifft die vergleichende Bestimmung der Veränderung des Membranpotentials von mindestens zwei Zellpopulationen, die mit unterschiedlichen Konzentrationen einer zu untersuchenden Substanz inkubiert werden.

Substanzen, die auf ihre Wirkung untersucht werden, werden als zu untersuchende Substanzen bezeichnet. Sustanzen, die eine Wirkung haben, d.h. die die Aktivität des hyperpolarisations-aktivierten Kationen-Kanals modulieren, können entweder Inhibitoren (inhibieren den Kanal und schwächen die Depolarisation oder verhindern sie ganz) oder Aktivatoren (aktivieren den Kanal und führen zu einer stärkeren oder schnelleren Depolarisation) des hyperpolarisations-aktivierten Kationen-Kanals sein.

Das Verfahren kann im Hochdurchsatzscreening (HTS) zur Identifizierung von Inhibitoren und/oder Aktivatoren eines hyperpolarisations-aktivierten Kationen-Kanals verwendet werden. Auf diese Weise identifizierte Substanzen können beispielsweise als pharmazeutische Wirkstoffe verwendet werden, zum Beispiel in einer pharmazeutisch verträglichen Form.

Ein Verfahren zur Herstellung eines Arzneimittels umfasst die Identifizierung einer Substanz, welche die Aktivität eines hyperpolarisations-aktivierten Kationen-Kanals inhibiert oder aktiviert, und schließlich die Mischung der identifizierten Substanz mit einem pharmazeutisch verträglichen Träger. Des weiteren umfasst ein Verfahren zur Herstellung eines Arzneimittels
a) die Identifizierung einer Substanz, die die Aktivität von hyperpolarisations-aktivierbaren Kationen-Kanälen moduliert;
b) die Herstellung der Substanz;
c) die Aufreinigung der Substanz; und
d) die Mischung der Substanz mit einem pharmazeutisch verträglichen Träger.

Gegenstand der Erfindung ist auch ein Test Kit zur Bestimmung, ob eine Substanz die Aktivität eines hyperpolarisations-aktivierbaren Kationen-Kanals moduliert, enthaltend
a) Zellen, die einen hyperpolarisations-aktivierten Kationen-Kanal überexprimieren;
b) einen isoosmolaren, Natriumionen-freien Puffer zur Hyperpolarisation der Zelle und
c) Reagenzien zur Detektion.

Als Maß für die Wirkung einer Substanz, wird die Veränderung des Membranpotentials der Zelle gemessen, vorzugsweise mit Hilfe eines potentialsensitiven Fluoreszenzfarbstoffs, z.B. mit einem Oxonolderivat, vorzugsweise 3-Bis-barbitursäure-oxonol.
Eine besondere Ausführungsform der Erfindung betrifft die Messung des Membranpotentials in einem Fluorescent Imaging Plate Reader (FLIPR).

### Abbildungen

Abb. 1: Die Abbildung zeigt den zeitlichen Verlauf der gemessenen Fluoreszenz als Mittelwert von jeweils 24 Wells. Um den zeitlichen Verlauf zwischen 60 und 290 Sekunden deutlicher erkennen zu können, wurde mittels der FLIPR-Software die in jedem Well gemessene Fluoreszenz (Summe der Helligkeitsstufen in einer festen und in allen Wells konstanten Zahl von Pixeln) zum Zeitpunkt 60 Sekunden (entspricht 40 Sekunden nach Na⁺-Zugabe) als "0" definiert. Die Kurven A und B wurden mit Zellen erhalten, die während des Anfärbens 10 µM Forskolin im Färbemedium enthielten; C und D sind Kurven von Zellen, die kein Forskolin bekamen. Bei A und C wurde im FLIPR NaCl auf eine Endkonzentration von 50 mM hinzugegeben, während B und D zusätzlich noch 8 mM CsCl erhielten, um den HCN2-Kanal zu hemmen. Mit der FLIPR-Software wurden die Werte der Kurve A (mit Forskolin; 50 mM NaCl + 8 mM CsCl) als Negativ-Kontrollen definiert.Die Kurven B, C und D stellen Fluoreszenzänderungen relativ zum Verlauf der Kurve A dar. Bei Kurve B (mit Forskolin; keine Hemmung mit CsCl) erkennt man gegenüber A eine deutliche Depolarisation. Zellen, die nicht mit Forskolin vorinkubiert wurden, aber bei Zugabe von NaCl gleichzeitig 8 mM CsCl erhielten (Kurve C), zeigen im Vergleich zu Kurve A einen abfallenden Verlauf. Daran ist zu erkennen, daß Forskolin in diesem Assay auch einen vom exprimierten HCN2-Kanal unabhängigen Einfluß auf das Membranpotential der Zellen hat. Gegenüber Kurve C zeigt auch Kurve D (kein Forskolin, keine Hemmung mit CsCl) wieder eine deutliche Depolarisation. Da der Abstand der Kurven A und B in dem Zeitraum ab 60 Sekunden aber stets größer ist als der Abstand der Kurven C und D, läßt sich erkennen, daß Forskolin den exprimierten HCN2-Kanal aktiviert.

Abb.2: Die Abbildung zeigt den Effekt von Zatebradine auf den Fluoreszenzverlauf. In dieser Abbildung haben alle Zellen während des Färbens 10 µM Forskolin erhalten. Kurve A zeigt den ungehemmten Verlauf (nur 50 mM NaCl), Kurve E den durch CsCl gehemmten Verlauf (50 mM NaCl + 8 mM CsCl), die Kurven B-D den Einfluß von Zatebradine: B: 12,5 µM, C: 25 µM und D: 50 µM. Die Kurven A und E stellen jeweils Mittelwerte der gemessenen Werte aus 12 Wells dar, die Kurven B, C und D aus 6 Wells. Die bei Zugabe von 8 mM CsCl gemessenen Werte (Kurve E) wurden als Negativ-Kontrollen definiert, so daß die Kurven A-D die relative Änderung zu Kurve E widerspiegeln. Man sieht deutlich, daß die Depolarisation, die nach Zugabe von NaCl eintritt, mit zunehmender Zatebradine-Konzentration abnimmt. Aus den im FLIPR gemessenen Fluoreszenzwerten konnte nach Export in Excel der IC₅₀-Wert für Zatebradine berechnet werden (26 µM).

### Beispiele

### Beispiel 1: Herstellung transfizierter Zellen

Das Plasmid pcDNA3-muHCN2 enthält die murine HCN2 (muHCN2) cDNA (Genbank Accession No: AJ225122) von Pos. 22-2812 (codierende Sequenz: Pos. 36-2627), einkloniert in die EcoR1- und Notl-Schnittstellen von pcDNA3, und wurde erhalten von M.Biel, TU München (Ludwig et al., 1998). Je 6 µg dieser Plasmid-DNA wurden zur Transfektion von CHO- bzw HEK293-Zellen verwendet. Zur Transfektion von CHO-Zellen bzw. HEK-Zellen wurde das LipofectAmine™Reagent der Firma LifeTechnologies (Gaithersburg, MD, USA) entsprechend dem Hersteller-Protokoll verwendet. 24 Stunden nach der Transfektion wurden die Zellen von Kulturschalen auf 75 cm² Zellkulturflaschen umgesetzt. 72 Stunden nach der Transfektion wurden die Zellen einer Selektion mit 400 µg/ml des Antibiotikums G418 (Calbiochem, Bad Soden, FRG) unterworfen. Nach einer zweiwöchigen Selektion wurden die überlebenden Zellen durch Trypsin-EDTA aus den Flaschen abgelöst, im Zellzähler Coulter Counter Z1 gezählt und so auf 96-Well Mikrotiterplatten ausgesät, daß statistisch 1 Zelle pro Well vorhanden sein sollte. Die Mikrotiterplatten wurden regelmäßig unter dem Mikroskop kontrolliert, und nur die Zellen aus den Wells, in denen nur eine Kolonie wuchs, wurden weiterkultiviert.
Aus diesen Zellen wurde mithilfe von QlAshredder und RNeasy-Kit der Firma Qiagen (Hilden, FRG). totale RNA isoliert. Diese totale RNA wurde mittels RT-PCR auf Expression des muHCN2 untersucht (Primer 1): 5'- GCCAATACCAGGAGAAG -3' [SEQ ID NO. 7], entspricht Pos. 1354-1370 in AJ225122, und Primer 2: 5'-TGAGTAGAGGCGACAGTAG -3' [SEQ ID NO. 8], entspricht Pos. 1829-1811 in AJ225122; erwartete RT-PCR Bande: 476 bp.

### Beispiel 2: Patch Clamp Untersuchung der Zellen

Mit der Patch-Clamp-Methode wurden die Zellen mit nachweisbarer mRNA-Expression elektrophysiologisch als Ganzzellableitung auf funktionale Expression des muHCN2 untersucht. Diese Methode ist bei Hamill et al. (1981) ausführlich beschrieben. Die Zellen wurden auf ein Haltepotential von -40 mV geklemmt. Von diesem Haltepotential ausgehend wurden die Ionenkanäle durch einen Spannungsprung auf -140 mV für die Dauer von einer Sekunde aktiviert. Die Stromamplitude wurde am Ende dieses Pulses bestimmt. Bei den transfizierten HEK-Zellen wurden einige mit Strömen um 1 nA gefunden, für die sich im FLIPR wegen störender endogener Ströme aber kein Assay aufbauen ließ.

Bei den HEK-Zellen zeigte sich aber deutlich, daß nur in Zellen mit starker mRNA-Expression ein funktional aktiver HCN2-Kanal nachweisbar war. Bei den CHO-Zellen bestätigte sich die Korrelation zwischen mRNA-Expression und Funktion. Generell war die mRNA-Expression in den HEK-Zellen etwa um einen Faktor drei besser als in den CHO-Zellen. In den Patch-Clamp Untersuchungen konnte bei einigen Zellen einer der am stärksten exprimierenden CHO-Zellinien ein schwacher Strom nachgewiesen werden.

### Beispiel 3: Herstellung doppelt transfizierter Zellen

Da die funktionale Expression streng mit der mRNA-Expression zu korrelieren schien, wurde eine zweite Transfektion mit der muHCN2 cDNA durchgeführt, die zuvor in die EcoRI- und Notl-Stelle des Vektors pcDNA3.1 (+)zeo einkloniert wurde. Nach zweiwöchiger Selektion mit G418 und Zeocin (Invitrogen, Groningen, NL) wurden, wie in Beispiel 1 beschrieben, einzelne Zellklone isoliert. Nach Isolierung der totalen RNA aus diesen Zellen wurde zunächst eine RT-PCR mit der in Beispiel 1 genannten Primern durchgeführt und anschließend eine RT-PCR mit den folgenden Primern, die eine Region umfassen, die das 3'-Ende der codierenden Sequenz von muHAC1 enthält (Primer 3: 5'- AGTGGCCTCGACCCACTGGACTCT -3' [SEQ ID NO. 9], entspricht Pos. 2553-2576 in AJ225122, und Primer 4: 5'- CCGCCTCCTAAGCTACCTACGTCCC -3' [SEQ ID NO. 10], entspricht Pos. 2725-2701 in AJ225122).

Einige dieser doppelt transfizierten Zellen zeigten sowohl in der RT-PCR als auch bei der Patch-Clamp-Analyse eine deutlich stärkere Expression als die Zellen, die nur einmal transfiziert worden waren. Elektrophysiologisch wurden Ströme bis zu 11 nA gemessen. Diese Zellen wurden zum Aufbau eines FLIPR-Assays für HCN2 verwendet.

### Beispiel 4: Aufbau eines FLIPR-Assays für HCN-Kanäle

Die auf Mikrotiterplatten ausgesäten Zellen werden in einem isoosmolaren Puffer gewaschen, bei dem NaCl durch Cholinchlorid ersetzt ist. Dieser Waschpuffer enthält aber noch 5 mM KCI, da extrazelluläres K⁺ für die HCN-Aktivität von Bedeutung ist (Biel et al. 1999). Dieser Waschpuffer, der zur Hyperpolarisation der HCN-Zellen dient, enthält gleichzeitig 5 µM DIBAC₄(3) zur Messung von Membranpotentialänderungen im FLIPR. Durch das Entfernen des extrazellulären Na⁺ werden die Zellen hyperpolarisiert, der HCN-Kanal also aktiviert. Er kann aber nicht zu einer Depolarisation der Zellen führen, da entsprechende Konzentrationsgradienten der von HCN-Kanälen transportierten Ionen Na⁺ oder K⁺ fehlen. Ein aktivierter HAC könnte jetzt höchstens zu einer stärkeren Hyperpolarisation führen. Dies spiegelt sich darin wieder, daß bei HAC-Zellen die im FLIPR gemessene Ausgangsfluoreszenz bei 10 µM Forskolin niedriger liegt als ohne Forskolin während es bei untransfizierten Zellen keinen Unterschied gibt.

Da die DiBAC₄(3)-Fluoreszenz empfindlich auf Temperaturschwankungen reagieren kann, wird nach einer Inkubation von 30 Minuten bei 37°C möglichst schnell gemessen; durch eine Abkühlung der Färbelösung kann das Meßergebnis verfälscht werden. Vorzugsweise wird vor Beginn der Messung eine fünfminütige Temperierungsphase im FLIPR durchgeführt.

Vorzugsweise werden die zu testenden Substanzen 10-fach konzentriert zu den DiBAC₄(3)-gefärbten Zellen zugegeben.

Im FLIPR wird Na⁺ zu den Zellen gegeben, so daß der aktivierte HCN (nach einigen Sekunden, in denen es zu Mischungseffekten kommt) ab etwa 15 Sekunden nach Na⁺-Zugabe zu einer Depolarisation der Zellen führt, die durch Zunahme der Fluoreszenz sichtbar wird. Entscheidend für das Auffinden von HCN-Modulatoren ist ein deutlicher Unterschied zwischen Zellen mit aktivem HCN-Kanal (nur Na⁺-Zugabe) und Zellen mit blockiertem HCN-Kanal (Na⁺ + 8mM CsCl). Eine Aktivierung des HCN-Kanals durch Vorinkubation mit 10 µM Forskolin vergrößert den Abstand des ungehemmten 100%-Wertes vom gehemmten 0%-Wert deutlich (s. Abb. 1). Im Vergleich zu den Kontrollwerten läßt sich erkennen, ob eine zu testende Substanz ein Aktivator (schnellere oder stärkere Depolarisation) oder ein Inhibitor ist (verlangsamte oder verhinderte Depolarisation, s. Abb. 2: Effekt von Zatebradine).

### Beispiel 5: Bestimmung des IC50-Wertes für einen HCN2-Blocker

An den transfizierten HCN-Zellen wurde die Wirkung verschiedener Konzentrationen der als l_{f}-Blockers bekannten Substanz Zatebradine untersucht (s. Abb. 2). Aus der relativen Veränderung der Fluoreszenz ab dem Zeitpunkt 60 Sekunden wurde die Hemmung durch Zatebradine berechnet. Für jede Konzentration des Hemmers wurde der Mittelwert von jeweils 6 Wells der Mikrotiterplatte bestimmt. Aus diesen Werten wurde für Zatebradine ein IC50-Wert von 26 µM berechnet, der gut mit dem elektrophysiologisch an den gleichen Zellen bestimmten Wert von 31 µM übereinstimmt.

### Beispiel 6: Verwendung des "FLIPR Membrane Assay Kit" (Molecular Devices, Sunnyvale, USA):

Am Vortag ausgesäte Zellen werden wie bisher dreimal mit jeweils 400 µl Waschpuffer pro Well gewaschen. Dabei wird das Restvolumen, das nach dem letzten Waschschritt über den Zellen stehen bleibt, jetzt jedoch je nach den gewünschten Konzentrationen an Na⁺ und Cs⁺ gewählt. Der Farbstoff in Waschpuffer wird hinzugegeben und die Zellen mit Farbstoff werden 30 Minuten bei Raumtemperatur, oder bei 37°C inkubiert, bevorzugt aber bei Raumtemperatur.

Im FLIPR wird anschließend die Depolarisation durch Zugabe von Na⁺ induziert und in einigen Kontroll-Wells durch gleichzeitige Zugabe von Cs⁺ wieder gehemmt.

Da eine Erhöhung der Ionenstärke bei dem Farbstoff von Molecular Devices zu Veränderung der Fluoreszenz führen kann, muss sichergestellt werden, dass sich in allen Wells einer Mikrotiterplatte die Ionenstärke im gleichen Maße verändert. Wann immer es die erwünschten Endkonzentration an Natrium- oder Cäsiumionen zulassen, wird versucht, die Osmolarität nicht zu verändern. Um die gewünschten Konzentrationen von Na+ und Cs+ einzustellen, werden neben dem Waschpuffer noch zwei weitere Puffer verwendet, die statt 140 mM Cholinchlorid 140 mM NaCl (Natriumpuffer) bzw. 140 mM CsCl (Cäsiumpuffer) enthalten.

Molecular Devices gibt für das Messen mit dem "FLIPR Membrane Potential Assay Kit" folgendes Grundprotokoll für 96-Well-Mikrotiterplatten an (384 Wells in Klammern): Die Zellen werden am Tag vor der Messung in 100 µl (25 µl) Medium ausgesät. Nach Zugabe von 100 µl (25 µl) Farbstoff und einer 30minütigen Inkubation bei Raumtemperatur oder bei 37°C; im FLIPR Zugabe von 50 µl (25 µl) der zu testenden Substanzen im passenden Puffer.

Mit den von Molecular Devices angegebenen Volumina lässt sich ohne Änderung der Ionenstärke in 96-Well-Platten eine Maximalkonzentration von 28 mM für Na⁺ + Cs⁺ erzielen, in 384-Well-Platten eine von 46,7 mM.
Da diese Konzentration besonders in den 96-Well-Platten zu niedrig sind für eine optimale Aktivität der hyperpolarisations-aktivierten Kationen-Kanäle, werden unterschiedliche Volumina für die einzelnen Schritte ausprobiert.
Es hat sich gezeigt, dass die Farbstoffkonzentration gegenüber dem von Molecular Devices angegebenem Protokoll halbiert werden kann.
In 96-Well-Platten erzielt man mit folgenden Volumina noch gute Ergebnisse:
45 µl Überstand von Waschpuffer über den Zellen, 60 µl Farbstoff in Waschpuffer, 195 µl Zugabevolumen im FLIPR.
Ein derartig hohes Zugabevolumen ermöglicht eine Maximalkonzentration für Na⁺ + Cs⁺ von 91 mM, d.h. bei 8-10 mM CsCl kann die Endkonzentration an NaCl 81-83 mM betragen. Für 80 mM Na⁺ und 8 mM Cs⁺ benötigt man bezogen auf 195 µl Zugabevolumen 6,43 µl Waschpuffer, 171,43 µl Natriumpuffer und 17,14 µl Cäsiumpuffer.

### Material und Methoden:

1. Lösungen und Puffer für die Messung mit DiBAC₄(3)
   A: DiBAC₄(3) Bis-(1,3-dibutylbarbitursäure)-trimethin oxonol Fa. Molecular Probes, Cat.No. B-438, MW: 516,64 g/mol
      In DMSO wird eine 10 mM Stammlösung von DIBAC₄(3) angesetzt (25 mg DIBAC₄(3)/ 4,838 ml DMSO). Aliquots dieser Stammlösung werden bei -20°C gelagert. Endkonzentration bei Färbung und Zugabe: 5 µM
   B: Forskolin MW: 410,5g/mol Endkonzentration bei Färbung: 10 µM Aliquots einer 10 mM Stammlösung in DMSO werden bei -20°C gelagert
   C: Waschpuffer: (140 mM Cholinchlorid, 5 mM KCI, 1 mM CaCl₂, 1 mM MgCl₂, 10 mM HEPES, 5 mM Glucose, mit 1 M KOH auf pH 7,4 einstellen)
   D: Presoak-Lösung zum Absättigen der Pipettenspitzen:
      wie Waschpuffer + 10 µM DIBAC₄(3)
      Diese Lösung wird nur für die Presoakplatte verwendet!
   E: Färbelösung: Doppelt konzentriert, d.h. Waschpuffer + 10 µM DIBAC₄(3) + 20 µM Forskolin.
   F: 10 fach konzentrierte Lösung für die Zugabeplatte: 500 mM NaCl in H₂O + 5 µM DIBAC
      Alle Substanzen werden 10-fach konzentriert in dieser Lösung angesetzt.
      Positiv-Kontrolle (Endkonzentration): 50 mM NaCl
      Negativ-Kontrolle (Endkonzentration): 50 mM NaCl + 8 mM CsCl
2. Lösungen und Puffer für das Messen mit dem "FLIPR Membrane Potential Assay Kit" von Molecular Devices
   A: "FLIPR Membrane Potential Assay Kit",
      Fa. Molecular Probes, Cat No. R8034
   B: Waschpuffer: (140 mM Cholinchlorid, 5 mM KCI, 1 mM CaCl₂, 1 mM MgCl₂, 10 mM HEPES, 5 mM Glucose, mit 1 M KOH auf pH 7,4 einstellen)
   C: Färbepuffer: (Inhalt von einem der "reagent vials" des "FLIPR Membrane Potential Assay Kits" in 10 ml Waschpuffer)
   D: Natriumpuffer: (140 mM NaCl, 5 mM KCI, 1 mM CaCl₂, 1 mM MgCl₂, 10 mM HEPES, 5 mM Glucose, mit 1 M KOH auf pH 7,4 einstellen)
   E: Cäsiumpuffer: (140mM CsCl, 5 mM KCI, 1 mM CaCl₂, 1 mM MgCl₂, 10 mM HEPES, 5 mM Glucose, mit 1 M KOH auf pH 7,4 einstellen)
3. Zellkulturarbeiten:
   Die mit muHCN2 transfizierten CHO-Zellen werden am Vortag mit einer Dichte von 35.000 Zellen/Well in jeweils 200µl Vollmedium auf schwarze 96-Well Mikrotiterplatten ausgesät. Über Nacht werden die Zellen bei 37°C und 5% CO₂ inkubiert.
4. Färbung mit DiBAC₄(3) und Messen im FLIPR:
   Vor der Färbung werden die Zellen im Zellwasher dreimal mit 400µl Waschpuffer gewaschen. Nach dem letzten Waschschritt bleibt ein Restvolumen von 90µl Waschpuffer/Well auf den Zellen stehen.
   Die gewaschenen Zellen (mit 90µl Waschpuffer/Well ) werden mit jeweils 90µl Färbelösung/Well für 30 Minuten bei 37°C im CO₂-Brutschrank inkubiert. Nach dieser Inkubationszeit wird die Zellplatte im FLIPR bei ca. 37°C (vorgegebene Temperatureinstellungen des FLIPR-Herstellers für Membranpotentialmessungen mit DiBAC₄(3)) entweder sofort oder nach einer fünfminütigen Temperierungsphase gemessen.
   Der Snapshot (anfängliche Fluoreszenz vor Beginn der Messung) sollte bei einem Durchschnittswert von etwa 35.000 Einheiten liegen. Im Maximum kann der FLIPR bis zu etwa 65.000 Einheiten auflösen.
   Beim Start des Programmes werden zunächst die Pipettenspitzen durch Eintauchen in Presoak-Lösung mit DiBAC₄(3) abgesättigt. Nach diesem Schritt beginnt die eigentliche Messung mit der ersten Aufnahme (t=0 Sekunden). Da DiBAC₄(3) ein langsam reagierender Farbstoff ist, reicht es, wenn die Fluoreszenz in den Wells der Mikrotiterplatte alle 5 Sekunden bestimmt wird. Nach etwa 20 Sekunden werden die Substanzen, die in der Zugabeplatte 10-fach konzentriert vorliegen, mittels des Pipettors gleichzeitig in die Mikrotiterplatte gegeben. Da das Volumen nach der Färbung 180µl beträgt, werden jeweils 20µl / Well hinzugegeben. Die Messung der Fluoreszenz kann nach etwa 5 Minuten beendet werden. Zur Auswertung wird die Fluoreszenzänderung in dem Zeitbereich betrachtet, in dem sie linear verläuft und in dem sich ungehemmte HCN2-transfizierte Zellen deutlich von gehemmten Zellen unterscheiden.
5. Färben mit dem "FLIPR Membrane Potential Assay Kit" und Messen im FLIPR Vor der Färbung werden die Zellen im Zellwasher dreimal mit 400µl Waschpuffer gewaschen. Nach dem letzten Waschschritt bleibt ein Restvolumen von 45-90 µl Waschpuffer/Well auf den Zellen stehen.
   Nach Zugabe der Farbstofflösung (Volumen abhängig von den gewünschten Endkonzentrationen) wird für 30 Minuten bei Raumtemperatur (bevorzugt) oder bei 37°C im CO₂-Brutschrank inkubiert. Nach dieser Inkubationszeit wird die Zellplatte im FLIPR bei Raumtemperatur gemessen.
   Der Snapshot (anfängliche Fluoreszenz vor Beginn der Messung) kann bei dem "FLIPR Membrane Potential Assay Kit" niedriger liegen als bei der Messung mit DiBAC₄(3), da der Assay-Kit sensitiver auf Änderungen des Membranpotentials reagiert als DiBAC₄(3). Wegen der größeren Sensitivität, die erzielt werden kann, sollte, wann immer möglich (FLIPRII), mit einem Emmissionsfilter gemessen werden, der für Licht oberhalb von 550 nm durchlässig ist. Aber auch mit dem Standardfilter, der zwischen 510 und 580 nm durchlässig ist, lässt sich messen.
   Mit dem Start des Programms (t=0) wird im FLIPR zunächst einige Male die Fluoreszenz aller Wells der Platte bestimmt, ehe die Depolarisation nach etwa 20 Sekunden durch Zugabe von Natriumionen gestartet wird. Dabei wird die Zugabelösung in jedem Fall aus den drei Puffern (Waschpuffer, Natriumpuffer bzw. Cäsiumpuffer) so gemischt, daß ihre Zugabe entweder zu keiner oder zu einer in allen Wells gleichen Änderung der Osmolarität führt. Die Messung der Fluoreszenz kann nach etwa 5 Minuten beendet werden. Als Negativkontrolle dienen die Wells, in denen neben Na⁺ zusätzlich noch 8 mM Cs⁺ zur völligen Blockade des HCN-Kanals zugegeben wurde. Zieht man diese Werte von den anderen ab, so ergibt sich ein gutes Maß für die Aktivität des HCN-Kanals unter dem Einfluss der zu untersuchenden Substanz. Zur Auswertung wird die Fluoreszenzänderung in dem Zeitbereich betrachtet, in dem sie linear verläuft und in dem sich ungehemmte HCN2-transfizierte Zellen deutlich von gehemmten Zellen unterscheiden.

### Literatur:

Biel M., Ludwig A., Zong X., Hofmann R. (1999) Hyperpolarization-activated cation channels: A multigene family. Rev. Physiol. Biochem. Pharmacol. 136: 165-181
Hamill O.P., Marty A., Neher E., Sakmann B., Sigworth F.J. (1981) Improved patch-clamp techniques for high-resolution current recording from cells and cell-free membrane patches. Pflügers Arch. 391: 85-100
Ludwig A., Zong X., Jeglitsch M., Hofmann F., Biel M. (1998) A family of hyperpolarization-activated mammalian cation channels. Nature 393: 587-591
Ludwig A., Zong X., Stieber J., Hullin R., Hofmann R., Biel M. (1999) Two pacemaker channels from heart with profoundly different activation kinetics. EMBO J. 18: 2323-2329
Reiffen A., Eberlein W., Müller P., Psiorz M., Noll K., Heider J., Lillie C., Kobinger W., Luger P. (1990) Specific bradycardiac agents. 1. Chemistry, pharmacology, and structure-activity relationships of substituted benzazepinones, a new class of compounds exerting antiischemic properties. J. Med. Chem. 33: 1496-1504.

**Tabelle 1: SEQ ID NO. 1 Proteinsequenz von huHCN2**

| Zugangsnummer: AAC28444 |
|---|
| |

**Tabelle 2: SEQ ID NO. 2 Nukleotidsequenz von huHCN2**

| Zugangsnummer: AF065164 |
|---|
| |
| |

**Tabelle 3: Proteinsequenz von huHCN4**

| Zugangsnummer: HSA132429 |
|---|
| |
| |

**Tabelle 4: SEQ ID NO. 4 Nukleotidsequenz von huHCN4**

| Zugangsnummer: HSA132429 |
|---|
| |
| |

**Tabelle 5: Proteinsequenz von muHCN2**

| Zugangsnummer: CAA12406 |
|---|
| |

**Tabelle 6: SEQ ID NO. 6 Nukleotidsequenz von muHCN2**

| Zugangsnummer: MMJ225122 |
|---|
| |
| |

**Tabelle 7: Verwendete Abkürzungen**

| | |
|---|---|
| AKT | (Arabidopsis thaliana K+-Transport) |
| cAMP | (cyclic adenosine monophosphate) zyklisches Adenosinmonophosphat |
| CHO | (chinese hamster ovary); Zellinie aus Ovarien des chinesischen Hamsters |
| EDTA | (ethylenediamine tetraacetic acid) Ethylendiamintetraessigsäure |
| FLIPR | (fluorescence imaging plate reader) |
| HAC | (hyperpolarization-activated cation channel) hyperpolarisations-aktivierter Kationen-Kanal; dieser Name wurde von einigen Arbeitsgruppen benutzt |
| HCN | (hyperpolarization-activated cyclic nucleotide gated cation channel); dies ist die neue, allgemein akzeptierte Bezeichnung |
| HEK | (human embryonic kidney); Zellinie aus menschlichen embryonalen Nierenzellen |
| HEPES | N-2-Hydroxyethylpiperazin-N'-2-ethansulfonsäure |
| HTS | (high throughput screening) Hochdurchsatz-Screening |
| KAT | (K+ channel from Arabidopsis thaliana) |

## Patentansprüche

1. Verfahren zur Identifizierung von Substanzen, die die Aktivität von hyperpolarisations-aktivierten Kationen-Kanälen modulieren, wobei
a) Zellen, die einen hyperpolarisations-aktivierten Kationen-Kanal exprimieren, verwendet werden;
b) die Zellen in Anwesenheit eines potentialsensitiven Fluoreszenzfarbstoffes mit einem isoosmolaren, Natriumionen-freien Puffer hyperpolarisiert werden; und
c) die Veränderung des Membranpotentials der Zellen nach gleichzeitiger Gabe von Natriumionen und der zu untersuchenden Substanz registriert wird.

2. Verfahren zur Identifizierung von Substanzen, die die Aktivität von hyperpolarisations-aktivierten Kationen-Kanälen modulieren, wobei
a) Zellen, die einen hyperpolarisations-aktivierten Kationen-Kanal exprimieren, verwendet werden;
b) die Zellen in Anwesenheit eines potentialsensitiven Fluoreszenzfarbstoffes mit einem isoosmolaren, Natriumionen-freien Puffer hyperpolarisiert werden;
c) die Zellen mit einer zu untersuchenden Substanz inkubiert werden; und
d) die Veränderung des Membranpotentials der Zellen nach Zugabe von Natriumionen registriert wird.

3. Verfahren nach einem oder mehreren der Ansprüche 1 und 2, wobei der isoosmolare, Natriumionen-freie Puffer ein Kaliumsalz enthält.

4. Verfahren nach einem oder mehreren der Ansprüche 1 bis 3, wobei der isoosmolare, Natriumionen-freie Puffer mindestens 0,8 mM K⁺ enthält.

5. Verfahren nach einem oder mehreren der Ansprüche 1 bis 4, wobei der isoosmolare, Natriumionen-freie Puffer mindestens 5 mM K⁺ enthält.

6. Verfahren nach einem oder mehreren der Ansprüche 1 bis 5, wobei der isomolare, Natriumionen-freie Puffer Cholinchlorid oder N-Methyl-D-glucamin enthält.

7. Verfahren nach einem oder mehreren der Ansprüche 1 bis 6, wobei der isomolare, Natriumionen-freie Puffer einen potentialsensitiven Farbstoff enthält.

8. Verfahren nach Anspruch 7, wobei der potentialsensitive Farbstoff ein Fluoreszenzfarbstoff ist.

9. Verfahren nach einem oder mehreren der Ansprüche 7 und 8, wobei der potentialsensitive Farbstoff ein Oxonolderivat ist.

10. Verfahren nach Anspruch 9, wobei das Oxonolderivat ein 3-Bis-barbitursäure-oxonol ist.

11. Verfahren nach Anspruch 10, wobei das 3-Bis-barbitursäure-oxonol Bis-(1,3-dibutylbarbitursäure)-trimethin oxonol, Bis-(1,3-diethylthiobarbitursäure)-trimethin oxonol und/oder Bis-(1,3-dibutylbarbitursäure)-pentamethin oxonol ist.

12. Verfahren nach einem oder mehreren der Ansprüche 1 bis 11, wobei Zellen verwendet werden, deren intrazelluläre cAMP Konzentration erhöht ist.

13. Verfahren nach Anspruch 12, wobei die intrazelluläre cAMP Konzentration durch Zugabe von Dibutyryl-cAMP oder 8-Bromo-cAMP erhöht wird.

14. Verfahren nach Anspruch 12, wobei die intrazelluläre cAMP Konzentration durch Zugabe eines Aktivators der Adenylatcyclase erhöht wird.

15. Verfahren nach Anspruch 12, wobei die intrazelluläre cAMP Konzentration durch Zugabe von Forskolin erhöht wird.

16. Verfahren nach Anspruch 13, wobei die intrazelluläre cAMP Konzentration durch Zugabe von 1 µM bis 100 µM Forskolin erhöht wird.

17. Verfahren nach Anspruch 12, wobei die intrazelluläre cAMP Konzentration durch Zugabe von Liganden von Rezeptoren erhöht wird.

18. Verfahren nach einem oder mehreren der Ansprüche 1 bis 17, wobei der hyperpolarisations-aktivierte Kationen-Kanal ein HCN1, HCN2, HCN3, HCN4, KAT1 oder ein Heteromultimeres dieser Kanäle ist.

19. Verfahren nach einem oder mehreren der Ansprüche 1 bis 18, wobei der hyperpolarisations-aktivierte Kationen-Kanal ein humaner hyperpolarisationsaktivierbarer Kationen-Kanal ist.

20. Verfahren nach einem oder mehreren der Ansprüche 1 bis 19, wobei die Zellen Säugerzellen sind.

21. Verfahren nach einem oder mehreren der Ansprüche 1 bis 20, wobei die Zellen CHO oder HEK Zellen sind.

22. Verfahren nach einem oder mehreren der Ansprüche 1 bis 21, wobei die Zellen mit einem Plasmid, das die cDNA eines hyperpolarisations-aktivierten Kationen-Kanals enthält, transfiziert werden.

23. Verfahren nach einem oder mehreren der Ansprüche 1 bis 22, wobei die Zellen mit einem zweiten Plasmid, das die cDNA desselben hyperpolarisations-aktivierten Kationen-Kanals enthält, transfiziert werden.

24. Verfahren nach einem oder mehreren der Ansprüche 1 bis 22, wobei die Zellen mit einem zweiten Plasmid, das die cDNA eines anderen hyperpolarisations-aktivierten Kationen-Kanals enthält, transfiziert werden, so daß sich Heteromultimere HCN-Kanäle bilden können.

25. Verfahren nach einem oder mehreren der Ansprüche 1 bis 22, wobei die Zellen mit einem Plasmid transfiziert werden, das eine synthetische cDNA enthält, bei der die Untereinheiten aus Teilen verschiedener Kanäle bzw. HCN-Kanäle zusammengesetzt werden.

26. Verfahren nach einem oder mehreren der Ansprüche 1 bis 25, wobei die Veränderung des Membranpotentials mit Hilfe eines potentialsensitiven Fluoreszenzfarbstoffs gemessen wird.

27. Verfahren nach Anspruch 26, wobei der potentialsensitive Fluoreszenzfarbstoff ein Oxonolderivat, vorzugsweise 3-Bis-barbitursäure-oxonol ist.

28. Verfahren nach einem oder mehreren der Ansprüche 1 bis 27, wobei die Messung in einem Fluorescent Imaging Plate Reader durchgeführt wird.

29. Verfahren nach einem oder mehreren der Ansprüche 1 bis 28, wobei die Veränderung des Membranpotentials von mindestens zwei Zellen vergleichend bestimmt wird.

30. Verwendung eines Verfahrens nach einem der Ansprüche 1 bis 29 im Hochdurchsatz-Screening zur Identifizierung von Inhibitoren und/oder Aktivatoren eines hyperpolarisations-aktivierten Kationen-Kanals.

31. Test Kit zur Bestimmung, ob eine Substanz die Aktivität eines hyperpolarisations-aktivierten Kationen-Kanals moduliert, enthaltend
a) Zellen, die einen hyperpolarisations-aktivierten Kationen-Kanal überexprimieren;
b) einen isoosmolaren, Natriumionen-freien Puffer zur Hyperpolarisation der Zelle und
c) Reagenzien zur Detektion.

## Claims

1. A process for identifying substances which modulate the activity of hyperpolarization-activated cation channels, wherein
a) cells which express a hyperpolarization-activated cation channel are used;
b) the cells are hyperpolarized in the presence of a potential-sensitive fluorescent dye using an isoosmolar sodium-ion-free buffer; and
c) the change in the membrane potential of the cells following simultaneous addition of sodium ions and the substance to be examined is recorded.

2. A process for identifying substances which modulate the activity of hyperpolarization-activated cation channels, wherein
a) cells which express a hyperpolarization-activated cation channel are used;
b) the cells are hyperpolarized in the presence of a potential-sensitive fluorescent dye using an isoosmolar sodium-ion-free buffer;
c) the cells are incubated with a substance to be examined; and
d) the change in the membrane potential of the cells after addition of sodium ions is recorded.

3. The process as claimed in one or both of claims 1 and 2, wherein the isoosmolar sodium-ion-free buffer comprises a potassium salt.

4. The process as claimed in one or more of claims 1 to 3, wherein the isoosmolar sodium-ion-free buffer comprises at least 0.8 mM K⁺.

5. The process as claimed in one or more of claims 1 to 4, wherein the isoosmolar sodium-ion-free buffer comprises at least 5 mM K⁺.

6. The process as claimed in one or more of claims 1 to 5, wherein the isoosmolar sodium-ion-free buffer comprises choline chloride or N-methyl-D-glucamine.

7. The process as claimed in one or more of claims 1 to 6, wherein the isoosmolar sodium-ion-free buffer comprises a potential-sensitive dye.

8. The process as claimed in claim 7, wherein the potential-sensitive dye is a fluorescent dye.

9. The process as claimed in one or both of claims 7 and 8, wherein the potential-sensitive dye is an oxonol derivative.

10. The process as claimed in claim 9, wherein the oxonol derivative is a 3-bis-barbituric acid oxonol.

11. The process as claimed in claim 10, wherein the 3-bis-barbituric acid oxonol is bis-(1,3-dibutylbarbituric acid)trimethine oxonol, bis-(1,3-diethylthiobarbituric acid)trimethine oxonol and/or bis-(1,3-dibutylbarbituric acid)pentamethine oxonol.

12. The process as claimed in one or more of claims 1 to 11, where cells having an elevated intracellular cAMP concentration are used.

13. The process as claimed in claim 12, wherein the intracellular cAMP concentration is increased by addition of dibutyryl-cAMP or 8-bromo-cAMP.

14. The process as claimed in claim 12, wherein the intracellular cAMP concentration is increased by addition of an adenylate cyclase activator.

15. The process as claimed in claim 12, wherein the intracellular cAMP concentration is increased by addition of forskolin.

16. The process as claimed in claim 13, wherein the intracellular cAMP concentration is increased by addition of from 1 µM to 100 µm of forskolin.

17. The process as claimed in claim 12, wherein the intracellular cAMP concentration is increased by addition of receptor ligands.

18. The process as claimed in one or more of claims 1 to 17, wherein the hyperpolarization-activated cation channel is HCN1, HCN2, HCN3, HCN4, KAT1 or a heteromultimer of these channels.

19. The process as claimed in one or more of claims 1 to 18, wherein the hyperpolarization-activated cation channel is a human hyperpolarization-activated cation channel.

20. The process as claimed in one or more of claims 1 to 19, wherein the cells are mammalian cells.

21. The process as claimed in one or more of claims 1 to 20, wherein the cells are CHO or HEK cells.

22. The process as claimed in one or more of claims 1 to 21, wherein the cells are transfected with a plasmid which comprises the cDNA of a hyperpolarization-activated cation channel.

23. The process as claimed in one or more of claims 1 to 22, wherein the cells are transfected with a second plasmid which comprises the cDNA of the same hyperpolarization-activated cation channel.

24. The process as claimed in one or more of claims 1 to 22, wherein the cells are transfected with a second plasmid which comprises the cDNA of a different hyperpolarization-activated cation channel, such that heteromultimeric HCN channels can be formed.

25. The process as claimed in one or more of claims 1 to 22, wherein the cells are transfected with a plasmid which comprises synthetic cDNA, where the subunits are composed of parts of different channels or HCN channels.

26. The process as claimed in one or more of claims 1 to 25, wherein the change in the membrane potential is measured using a potential-sensitive fluorescent dye.

27. The process as claimed in claim 26, wherein the potential-sensitive fluorescent dye is an oxonol derivative, preferably 3-bis-barbituric acid oxonol.

28. The process as claimed in one or more of claims 1 to 27, wherein the measurement is carried out in a Fluorescent Imaging Plate Reader.

29. The process as claimed in one or more of claims 1 to 28, wherein the change of the membrane potential of at least two cells is compared.

30. The use of a process as claimed in any of claims 1 to 29 in high-throughput screening for identifying inhibitors and/or activators of a hyperpolarization-activated cation channel.

31. A test kit for determining whether a substance modulates the activity of a hyperpolarization-activated cation channel, which test kit comprises
a) cells which overexpress a hyperpolarization-activated cation channel;
b) an isoosmolar sodium-ion-free buffer for hyperpolarizing the cell and
c) reagents for detection.

## Revendications

1. Procédé pour l'identification de substances qui modulent l'activité de canaux cationiques activés par hyperpolarisation, dans lequel
a) on utilise des cellules qui expriment un canal activé par hyperpolarisation ;
b) les cellules sont hyperpolarisées avec un tampon iso-osmolaire, exempt d'ions sodium, en présence d'un colorant fluorescent sensible au potentiel ; et
c) on enregistre la variation du potentiel membranaire des cellules après apport simultané d'ions sodium et de la substance à étudier.

2. Procédé pour l'identification de substances qui modulent l'activité de canaux cationiques activés par hyperpolarisation, dans lequel
a) on utilise des cellules qui expriment un canal activé par hyperpolarisation ;
b) les cellules sont hyperpolarisées avec un tampon iso-osmolaire, exempt d'ions sodium, en présence d'un colorant fluorescent sensible au potentiel ;
c) on met les cellules à incuber avec une substance à étudier ; et
d) on enregistre la variation du potentiel membranaire des cellules après addition d'ions sodium.

3. Procédé selon une ou plusieurs des revendications 1 et 2, dans lequel le tampon iso-osmolaire exempt d'ions sodium contient un sel de potassium.

4. Procédé selon une ou plusieurs des revendications 1 à 3, dans lequel le tampon iso-osmolaire exempt d'ions sodium contient au moins 0,8 mM de K⁺.

5. Procédé selon une ou plusieurs des revendications 1 à 4, dans lequel le tampon iso-osmolaire exempt d'ions sodium contient au moins 5 mM de K⁺.

6. Procédé selon une ou plusieurs des revendications 1 à 5, dans lequel le tampon iso-osmolaire exempt d'ions sodium contient du chlorure de choline ou de la N-méthyl-D-glucamine.

7. Procédé selon une ou plusieurs des revendications 1 à 6, dans lequel le tampon iso-osmolaire exempt d'ions sodium contient un colorant sensible au potentiel.

8. Procédé selon la revendication 7, dans lequel le colorant sensible au potentiel est un colorant fluorescent.

9. Procédé selon une ou plusieurs des revendications 7 et 8, dans lequel le colorant sensible au potentiel est un dérivé d'oxonol.

10. Procédé selon la revendication 9, dans lequel le dérivé d'oxonol est un 3-bis-barbituryl-oxonol.

11. Procédé selon la revendication 10, dans lequel le 3-bis-barbituryl-oxonol est le bis(1,3-dibutylbarbituryl)triméthine-oxonol et/ou le bis(1,3-diéthylthiobarbituryl)triméthine-oxonol et/ou le bis(1,3-dibutylbarbityryl)pentaméthine-oxonol.

12. Procédé selon une ou plusieurs des revendications 1 à 11, dans lequel on utilise des cellules dont la teneur intracellulaire en cAMP est accrue.

13. Procédé selon la revendication 12, dans lequel la teneur intracellulaire en cAMP est accrue par addition de dibutyryl-cAMP ou de 8-bromo-cAMP.

14. Procédé selon la revendication 12, dans lequel la teneur intracellulaire en cAMP est accrue par addition d'un activateur de l'adénylate cyclase.

15. Procédé selon la revendication 12, dans lequel la teneur intracellulaire en cAMP est accrue par addition de forskoline.

16. Procédé selon la revendication 13, dans lequel la teneur intracellulaire en cAMP est accrue par addition de 1 µM à 100 µM de forskoline.

17. Procédé selon la revendication 12, dans lequel la teneur intracellulaire en cAMP est accrue par addition de ligands ou de récepteurs.

18. Procédé selon une ou plusieurs des revendications 1 à 17, dans lequel le canal cationique activé par hyperpolarisation est un canal HCN1, HCN2, HCN3, HCN4, KAT1 ou un hétéromultimère de ces canaux.

19. Procédé selon une ou plusieurs des revendications 1 à 18, dans lequel le canal cationique activé par hyperpolarisation est un canal cationique humain activable par hyperpolarisation.

20. Procédé selon une ou plusieurs des revendications 1 à 19, dans lequel les cellules sont des cellules mammaliennes .

21. Procédé selon une ou plusieurs des revendications 1 à 20, dans lequel les cellules sont des cellules CHO ou HEK.

22. Procédé selon une ou plusieurs des revendications 1 à 21, dans lequel les cellules sont transfectées par un plasmide qui contient l'ADNc d'un canal cationique activé par hyperpolarisation.

23. Procédé selon une ou plusieurs des revendications 1 à 22, dans lequel les cellules sont transfectées par un second plasmide qui contient l'ADNc du même canal cationique activé par hyperpolarisation.

24. Procédé selon une ou plusieurs des revendications 1 à 22, dans lequel les cellules sont transfectées par un second plasmide qui contient l'ADNc d'un autre canal cationique activé par hyperpolarisation, de sorte que des canaux HCN hétéromultimères peuvent se former.

25. Procédé selon une ou plusieurs des revendications 1 à 22, dans lequel les cellules sont transfectées par un plasmide qui contient un ADNc synthétique dans lequel sont réunies les sous-unités provenant de parties de canaux différents ou de canaux HCN.

26. Procédé selon une ou plusieurs des revendications 1 à 25, dans lequel la variation du potentiel membranaire est mesurée à l'aide d'un colorant fluorescent sensible au potentiel.

27. Procédé selon la revendication 26, dans lequel le colorant fluorescent sensible au potentiel est un dérivé d'oxonol, de préférence le 3-bis-barbituryl-oxonol.

28. Procédé selon une ou plusieurs des revendications 1 à 27, dans lequel on effectue la mesure dans un lecteur de plaques à imagerie fluorescente *Fluorescent Imaging Plate Reader.*

29. Procédé selon une ou plusieurs des revendications 1 à 28, dans lequel on détermine par comparaison la variation du potentiel membranaire d'au moins deux cellules.

30. Utilisation d'un procédé selon une ou plusieurs des revendications 1 à 29, dans le criblage à haut rendement pour l'identification d'inhibiteurs et/ou d'activateurs d'un canal cationique activé par hyperpolarisation.

31. Nécessaire d'essai pour déterminer si une substance module l'activité d'un canal cationique activé par hyperpolarisation, contenant
a) des cellules qui surexpriment un canal cationique activé par hyperpolarisation ;
b) un tampon iso-osmolaire exempt d'ions sodium, pour l'hyperpolarisation de la cellule et
c) des réactifs pour la détection.


### SEQUENZPROTOKOLL

<110> Aventis Pharma Deutschland GmbH
<120> Verfahren zur Identifizierung von Substanzen, die die Aktivität von hyperpolarisations-aktivierten Kationen-kanälen modulieren
<130> AVE D-2000/A006
<140> 10006309.8
   <141> 2000-02-12
<160> 10
<170> PatentIn Ver. 2.1
<210> 1
   <211> 889
   <212> PRT
   <213> Homo sapiens
<400> 1
   Met Asp Ala Arg Gly Gly Gly Gly Arg Pro Gly Glu Ser Pro Gly Ala
   1 5 10 15
   Ser Pro Thr Thr Gly Pro Pro Pro Pro Pro Pro Pro Arg Pro Pro Lys
   20 25 30
   Gln Gln Pro Pro Pro Pro Pro Pro Pro Ala Pro Pro Pro Gly Pro Gly
   35 40 45
   Pro Ala Pro Pro Gln His Pro Pro Arg Ala Glu Ala Leu Pro Pro Glu
   50 55 60
   Ala Ala Asp Glu Gly Gly Pro Arg Gly Arg Leu Arg Ser Arg Asp Ser
   65 70 75 80
   Ser Cys Gly Arg Pro Gly Thr Pro Gly Ala Ala Ser Thr Ala Lys Gly
   85 90 95
   Ser Pro Asn Gly Glu Cys Gly Arg Gly Glu Pro Gln Cys Ser Pro Ala
   100 105 110
   Gly Pro Glu Gly Pro Ala Arg Gly Pro Lys Val Ser Phe Ser Cys Arg
   115 120 125
   Gly Ala Ala Ser Gly Pro Ala Pro Gly Pro Gly Pro Ala Glu Glu Ala
       130                 135                 140
   Gly Ser Glu Glu Ala Gly Pro Ala Gly Glu Pro Arg Gly Ser Gln Ala
   145                 150                 155                 160
   Ser Phe Met Gln Arg Gln Phe Gly Ala Leu Leu Gln Pro Gly Val Asn
                   165                 170                 175
   Lys Phe Ser Leu Arg Met Phe Gly Ser Gln Lys Ala Val Glu Arg Glu
               180                 185                 190
   Gln Glu Arg Val Lys Ser Ala Gly Ala Trp Ile Ile His Pro Tyr Ser
           195                 200                 205
   Asp Phe Arg Phe Tyr Trp Asp Phe Thr Met Leu Leu Phe Met Val Gly
       210                 215                 220
   Asn Leu Ile Ile Ile Pro Val Gly Ile Thr Phe Phe Lys Asp Glu Thr
   225                 230                 235                 240
   Thr Ala Pro Trp Ile Val Phe Asn Val Val Ser Asp Thr Phe Phe Leu
                   245                 250                 255
   Met Asp Leu Val Leu Asn Phe Arg Thr Gly Ile Val Ile Glu Asp Asn
               260                 265                 270
   Thr Glu Ile Ile Leu Asp Pro Glu Lys Ile Lys Lys Lys Tyr Leu Arg
           275                 280                 285
   Thr Trp Phe Val Val Asp Phe Val Ser Ser Ile Pro Val Asp Tyr Ile
       290                 295                 300
   Phe Leu Ile Val Glu Lys Gly Ile Asp Ser Glu Val Tyr Lys Thr Ala
   305                 310                 315                 320
   Arg Ala Leu Arg Ile Val Arg Phe Thr Lys Ile Leu Ser Leu Leu Arg
                   325                 330                 335
   Leu Leu Arg Leu Ser Arg Leu Ile Arg Tyr Ile His Gln Trp Glu Glu
               340                 345                 350
   Ile Phe His Met Thr Tyr Asp Leu Ala Ser Ala Val Met Arg Ile Cys
           355                 360                 365
   Asn Leu Ile Ser Met Met Leu Leu Leu Cys His Trp Asp Gly Cys Leu
       370                 375                 380
   Gln Phe Leu Val Pro Met Leu Gln Asp Phe Pro Arg Asn Cys Trp Val
   385 390 395 400
   Ser Ile Asn Gly Met Val Asn His Ser Trp Ser Glu Leu Tyr Ser Phe
   405 410 415
   Ala Leu Phe Lys Ala Met Ser His Met Leu Cys Ile Gly Tyr Gly Arg
   420 425 430
   Gln Ala Pro Glu Ser Met Thr Asp Ile Trp Leu Thr Met Leu Ser Met
   435 440 445
   Ile Val Gly Ala Thr Cys Tyr Ala Met Phe Ile Gly His Ala Thr Ala
   450 455 460
   Leu Ile Gln Ser Leu Asp Ser Ser Arg Arg Gln Tyr Gln Glu Lys Tyr
   465 470 475 480
   Lys Gln Val Glu Gln Tyr Met Ser Phe His Lys Leu Pro Ala Asp Phe
   485 490 495
   Arg Gln Lys Ile His Asp Tyr Tyr Glu His Arg Tyr Gln Gly Lys Met
   500 505 510
   Phe Asp Glu Asp Ser Ile Leu Gly Glu Leu Asn Gly Pro Leu Arg Glu
   515 520 525
   Glu Ile Val Asn Phe Asn Cys Arg Lys Leu Val Ala Ser Met Pro Leu
   530 535 540
   Phe Ala Asn Ala Asp Pro Asn Phe Val Thr Ala Met Leu Thr Lys Leu
   545 550 555 560
   Lys Phe Glu Val Phe Gln Pro Gly Asp Tyr Ile Ile Arg Glu Gly Thr
   565 570 575
   Ile Gly Lys Lys Met Tyr Phe Ile Gln His Gly Val Val Ser Val Leu
   580 585 590
   Thr Lys Gly Asn Lys Glu Met Lys Leu Ser Asp Gly Ser Tyr Phe Gly
   595 600 605
   Glu Ile Cys Leu Leu Thr Arg Gly Arg Arg Thr Ala Ser Val Arg Ala
   610 615 620
   Asp Thr Tyr Cys Arg Leu Tyr Ser Leu Ser Val Asp Asn Phe Asn Glu
   625 630 635 640
   Val Leu Glu Glu Tyr Pro Met Met Arg Arg Ala Phe Glu Thr Val Ala
   645 650 655
   Ile Asp Arg Leu Asp Arg Ile Gly Lys Lys Asn Ser Ile Leu Leu His
   660 665 670
   Lys Val Gln His Asp Leu Asn Ser Gly Val Phe Asn Asn Gln Glu Asn
   675 680 685
   Ala Ile Ile Gln Glu Ile Val Lys Tyr Asp Arg Glu Met Val Gln Gln
   690 695 700
   Ala Glu Leu Gly Gln Arg Val Gly Leu Phe Pro Pro Pro Pro Pro Pro
   705 710 715 720
   Pro Gln Val Thr Ser Ala Ile Ala Thr Leu Gln Gln Ala Ala Ala Met
   725 730 735
   Ser Phe Cys Pro Gln Val Ala Arg Pro Leu Val Gly Pro Leu Ala Leu
   740 745 750
   Gly Ser Pro Arg Leu Val Arg Arg Pro Pro Pro Gly Pro Ala Pro Ala
   755 760 765
   Ala Ala Ser Pro Gly Pro Pro Pro Pro Ala Ser Pro Pro Gly Ala Pro
   770 775 780
   Ala Ser Pro Arg Ala Pro Arg Thr Ser Pro Tyr Gly Gly Leu Pro Ala
   785 790 795 800
   Ala Pro Leu Ala Gly Pro Ala Leu Pro Ala Arg Arg Leu Ser Arg Ala
   805 810 815
   Ser Arg Pro Leu Ser Ala Ser Gln Pro Ser Leu Pro His Gly Ala Pro
   820 825 830
   Gly Pro Ala Ala Ser Thr Arg Pro Ala Ser Ser Ser Thr Pro Arg Leu
   835 840 845
   Gly Pro Thr Pro Ala Ala Arg Ala Ala Ala Pro Ser Pro Asp Arg Arg
   850 855 860
   Asp Ser Ala Ser Pro Gly Ala Ala Gly Gly Leu Asp Pro Gln Asp Ser
   865 870 875 880
   Ala Arg Ser Arg Leu Ser Ser Asn Leu
   885
<210> 2
   <211> 3372
   <212> DNA
   <213> Homo sapiens
<400> 2
   cggctccgct ccgcactgcc cggcgccgcc tcgccatgga cgcgcgcggg ggcggcgggc 60
   ggcccgggga gagcccgggc gcgagcccca cgaccgggcc gccgccgccg ccgcccccgc 120
   gcccccccaa acagcagccg ccgccgccgc cgccgcccgc gccccccccg ggccccgggc 180
   ccgcgccccc ccagcacccg ccccgggccg aggcgttgcc cccggaggcg gcggatgagg 240
   gcggcccgcg gggccggctc cgcagccgcg acagctcgtg cggccgcccc ggcaccccgg 300
   gcgcggcgag cacggccaag ggcagcccga acggcgagtg cgggcgcggc gagccgcagt 360
   gcagccccgc ggggcccgag ggcccggcgc gggggcccaa ggtgtcgttc tcgtgccgcg 420
   gggcggcctc ggggcccgcg ccggggccgg ggccggcgga ggaggcgggc agcgaggagg 480
   cgggcccggc gggggagccg cgcggcagcc aggccagctt catgcagcgc cagttcggcg 540
   cgctcctgca gccgggcgtc aacaagttct cgctgcggat gttcggcagc cagaaggccg 600
   tggagcgcga gcaggagcgc gtcaagtcgg cgggggcctg gatcatccac ccgtacagcg 660
   acttcaggtt ctactgggac ttcaccatgc tgctgttcat ggtgggaaac ctcatcatca 720
   tcccagtggg catcaccttc ttcaaggatg agaccactgc cccgtggatc gtgttcaacg 780
   tggtctcgga caccttcttc ctcatggacc tggtgttgaa cttccgcacc ggcattgtga 840
   tcgaggacaa cacggagatc atcctggacc ccgagaagat caagaagaag tatctgcgca 900
   cgtggttcgt ggtggacttc gtgtcctcca tccccgtgga ctacatcttc cttatcgtgg 960
   agaagggcat tgactccgag gtctacaaga cggcacgcgc cctgcgcatc gtgcgcttca 1020
   ccaagatcct cagcctcctg cggctgctgc gcctctcacg cctgatccgc tacatccatc 1080
   agtgggagga gatcttccac atgacctatg acctggccag cgcggtgatg aggatctgca 1140
   atctcatcag catgatgctg ctgctctgcc actgggacgg ctgcctgcag ttcctggtgc 1200
   ctatgctgca ggacttcccg cgcaactgct gggtgtccat caatggcatg gtgaaccact 1260
   cgtggagtga actgtactcc ttcgcactct tcaaggccat gagccacatg ctgtgcatcg 1320
   ggtacggccg gcaggcgccc gagagcatga cggacatctg gctgaccatg ctcagcatga 1380
   ttgtgggtgc cacctgctac gccatgttca tcggccacgc cactgccctc atccagtcgc 1440
   tggactcctc gcggcgccag taccaggaga agtacaagca ggtggagcag tacatgtcct 1500
   tccacaagct gccagctgac ttccgccaga agatccacga ctactatgag caccgttacc 1560
   agggcaagat gtttgacgag gacagcatcc tgggcgagct caacgggccc ctgcgggagg 1620
   agatcgtcaa cttcaactgc cggaagctgg tggcctccat gccgctgttc gccaacgccg 1680
   accccaactt cgtcacggcc atgctgacca agctcaagtt cgaggtcttc cagccgggtg 1740
   actacatcat ccgcgaaggc accatcggga agaagatgta cttcatccag cacggcgtgg 1800
   tcagcgtgct cactaagggc aacaaggaga tgaagctgtc cgatggctcc tacttcgggg 1860
   agatctgcct gctcacccgg ggccgccgca cggcgagcgt gcgggctgac acctactgcc 1920
   gcctctattc gctgagcgtg gacaacttca acgaggtgct ggaggagtac cccatgatgc 1980
   ggcgcgcctt cgagacggtg gccatcgacc gcctggaccg catcggcaag aagaattcca 2040
   tcctcctgca caaggtgcag catgacctca actcgggcgt attcaacaac caggagaacg 2100
   ccatcatcca ggagatcgtc aagtacgacc gcgagatggt gcagcaggcc gagctgggtc 2160
   agcgcgtggg cctcttcccg ccgccgccgc cgccgccgca ggtcacctcg gccatcgcca 2220
   cgctgcagca ggcggcggcc atgagcttct gcccgcaggt ggcgcggccg ctcgtggggc 2280
   cgctggcgct cggctcgccg cgcctcgtgc gccgcccgcc cccggggccc gcacctgccg 2340
   ccgcctcacc cgggcccccg ccccccgcca gcccccgggg cgcgcccgcc agcccccggg 2400
   caccgcggac ctcgccctac ggcggcctgc ccgccgcccc ccttgctggg cccgccctgc 2460
   ccgcgcgccg cctgagccgc gcgtcgcgcc cactgtccgc ctcgcagccc tcgctgcctc 2520
   acggcgcccc cggccccgcg gcctccacac gcccggccag cagctccaca ccgcgcttgg 2580
   ggcccacgcc cgctgcccgg gccgccgcgc ccagcccgga ccgcagggac tcggcctcac 2640
   ccggcgccgc cggcggcctg gacccccagg actccgcgcg ctcgcgcctc tcgtccaact 2700
   tgtgaccctc gccgaccgcc ccgcgggccc aggcgggccg ggggcggggc cgtcatccag 2760
   accaaagcca tgccattgcg ctgccccggc cgccagtccg cccagaagcc atagacgaga 2820
   cgtaggtagc cgtagttgga cggacgggca gggccggcgg ggcagccccc tccgcgcccc 2880
   cggccgtccc ccctcatcgc cccgcgccca cccccatcgc ccctgccccc ggcggcggcc 2940
   tcgcgtgcga gggggctccc ttcacctcgg tgcctcagtt cccccagctg taagacaggg 3000
   acggggcggc ccagtggctg agaggagccg gctgtggagc cccgcccgcc ccccaccctc 3060
   taggtggccc ccgtccgagg aggatcgttt tctaagtgca atacttggcc cgccggcttc 3120
   ccgctgcccc catcgcgctc acgcaataac cggcccggcc cccgtccgcg cgcgtccccc 3180
   ggtgacctcg gggagcagca ccccgcctcc ctccagcact ggcaccgaga ggcaggcctg 3240
   gctgcgcagg gcgcgggggg gaggctgggg tcccgccgcc gtgatgaatg tactgacgag 3300
   ccgaggcagc agtgccccca ccgtggcccc ccacgcccca ttaaccccca cacccccatt 3360
   ccgcgcaata aa                                                     3372
<210> 3
   <211> 1203
   <212> PRT
   <213> Homo sapiens
<400> 3
<210> 4
   <211> 5065
   <212> DNA
   <213> Homo sapiens
<400> 4
<210> 5
   <211> 863
   <212> PRT
   <213> Murinae gen. sp.
<400> 5
   Met Asp Ala Arg Gly Gly Gly Gly Arg Pro Gly Asp Ser Pro Gly Thr
    1               5                   10                  15
   Thr Pro Ala Pro Gly Pro Pro Pro Pro Pro Pro Pro Pro Ala Pro Pro
               20                  25                  30
   Gln Pro Gln Pro Pro Pro Ala Pro Pro Pro Asn Pro Thr Thr Pro Ser
           35                  40                  45
   His Pro Glu Ser Ala Asp Glu Pro Gly Pro Arg Ala Arg Leu Cys Ser
       50                  55                  60
   Arg Asp Ser Ala Cys Thr Pro Gly Ala Ala Lys Gly Gly Ala Asn Gly
   65                  70                  75                  80
   Glu Cys Gly Arg Gly Glu Pro Gln Cys Ser Pro Glu Gly Pro Ala Arg
                   85                  90                  95
   Gly Pro Lys Val Ser Phe Ser Cys Arg Gly Ala Ala Ser Gly Pro Ser
               100                 105                 110
   Ala Ala Glu Glu Ala Gly Ser Glu Glu Ala Gly Pro Ala Gly Glu Pro
           115                 120                 125
   Arg Gly Ser Gln Ala Ser Phe Leu Gln Arg Gln Phe Gly Ala Leu Leu
       130                 135                 140
   Gln Pro Gly Val Asn Lys Phe Ser Leu Arg Met Phe Gly Ser Gln Lys
   145                 150                 155                 160
   Ala Val Glu Arg Glu Gln Glu Arg Val Lys Ser Ala Gly Ala Trp Ile
                   165                 170                 175
   Ile His Pro Tyr Ser Asp Phe Arg Phe Tyr Trp Asp Phe Thr Met Leu
               180                 185                 190
   Leu Phe Met Val Gly Asn Leu Ile Ile Ile Pro Val Gly Ile Thr Phe
           195                 200                 205
   Phe Lys Asp Glu Thr Thr Ala Pro Trp Ile Val Phe Asn Val Val Ser
       210                 215                 220
   Asp Thr Phe Phe Leu Met Asp Leu Val Leu Asn Phe Arg Thr Gly Ile
   225                 230                 235                 240
   Val Ile Glu Asp Asn Thr Glu Ile Ile Leu Asp Pro Glu Lys Ile Lys
                   245                 250                 255
   Lys Lys Tyr Leu Arg Thr Trp Phe Val Val Asp Phe Val Ser Ser Ile
               260                 265                 270
   Pro Val Asp Tyr Ile Phe Leu Ile Val Glu Lys Gly Ile Asp Ser Glu
           275                 280                 285
   Val Tyr Lys Thr Ala Arg Ala Leu Arg Ile Val Arg Phe Thr Lys Ile
       290                 295                 300
   Leu Ser Leu Leu Arg Leu Leu Arg Leu Ser Arg Leu Ile Arg Tyr Ile
   305                 310                 315                 320
   His Gln Trp Glu Glu Ile Phe His Met Thr Tyr Asp Leu Ala Ser Ala
                   325                 330                 335
   Val Met Arg Ile Cys Asn Leu Ile Ser Met Met Leu Leu Leu Cys His
               340                 345                 350
   Trp Asp Gly Cys Leu Gln Phe Leu Val Pro Met Leu Gln Asp Phe Pro
           355                 360                 365
   Ser Asp Cys Trp Val Ser Ile Asn Asn Met Val Asn His Ser Trp Ser
       370                 375                 380
   Glu Leu Tyr Ser Phe Ala Leu Phe Lys Ala Met Ser His Met Leu Cys
   385                 390                 395                 400
   Ile Gly Tyr Gly Arg Gln Ala Pro Glu Ser Met Thr Asp Ile Trp Leu
                   405                 410                 415
   Thr Met Leu Ser Met Ile Val Gly Ala Thr Cys Tyr Ala Met Phe Ile
   420 425 430
   Gly His Ala Thr Ala Leu Ile Gln Ser Leu Asp Ser Ser Arg Arg Gln
   435 440 445
   Tyr Gln Glu Lys Tyr Lys Gln Val Glu Gln Tyr Met Ser Phe His Lys
   450 455 460
   Leu Pro Ala Asp Phe Arg Gln Lys Ile His Asp Tyr Tyr Glu His Arg
   465 470 475 480
   Tyr Gln Gly Lys Met Phe Asp Glu Asp Ser Ile Leu Gly Glu Leu Asn
   485 490 495
   Gly Pro Leu Arg Glu Glu Ile Val Asn Phe Asn Cys Arg Lys Leu Val
   500 505 510
   Ala Ser Met Pro Leu Phe Ala Asn Ala Asp Pro Asn Phe Val Thr Ala
   515 520 525
   Met Leu Thr Lys Leu Lys Phe Glu Val Phe Gln Pro Gly Asp Tyr Ile
   530 535 540
   Ile Arg Glu Gly Thr Ile Gly Lys Lys Met Tyr Phe Ile Gln His Gly
   545 550 555 560
   Val Val Ser Val Leu Thr Lys Gly Asn Lys Glu Met Lys Leu Ser Asp
   565 570 575
   Gly Ser Tyr Phe Gly Glu Ile Cys Leu Leu Thr Arg Gly Arg Arg Thr
   580 585 590
   Ala Ser Val Arg Ala Asp Thr Tyr Cys Arg Leu Tyr Ser Leu Ser Val
   595 600 605
   Asp Asn Phe Asn Glu Val Leu Glu Glu Tyr Pro Met Met Arg Arg Ala
   610 615 620
   Phe Glu Thr Val Ala Ile Asp Arg Leu Asp Arg Ile Gly Lys Lys Asn
   625 630 635 640
   Ser Ile Leu Leu His Lys Val Gln His Asp Leu Ser Ser Gly Val Phe
   645 650 655
   Asn Asn Gln Glu Asn Ala Ile Ile Gln Glu Ile Val Lys Tyr Asp Arg
   660 665 670
   Glu Met Val Gln Gln Ala Glu Leu Gly Gln Arg Val Gly Leu Phe Pro
   675 680 685
   Pro Pro Pro Pro Pro Gln Val Thr Ser Ala Ile Ala Thr Leu Gln Gln
   690 695 700
   Ala Val Ala Met Ser Phe Cys Pro Gln Val Ala Arg Pro Leu Val Gly
   705 710 715 720
   Pro Leu Ala Leu Gly Ser Pro Arg Leu Val Arg Arg Ala Pro Pro Gly
   725 730 735
   Pro Leu Pro Pro Ala Ala Ser Pro Gly Pro Pro Ala Ala Ser Pro Pro
   740 745 750
   Ala Ala Pro Ser Ser Pro Arg Ala Pro Arg Thr Ser Pro Tyr Gly Val
   755 760 765
   Pro Gly Ser Pro Ala Thr Arg Val Gly Pro Ala Leu Pro Ala Arg Arg
   770 775 780
   Leu Ser Arg Ala Ser Arg Pro Leu Ser Ala Ser Gln Pro Ser Leu Pro
   785 790 795 800
   His Gly Val Pro Ala Pro Ser Pro Ala Ala Ser Ala Arg Pro Ala Ser
   805 810 815
   Ser Ser Thr Pro Arg Leu Gly Pro Ala Pro Thr Ala Arg Thr Ala Ala
   820 825 830
   Pro Ser Pro Asp Arg Arg Asp Ser Ala Ser Pro Gly Ala Ala Ser Gly
   835 840 845
   Leu Asp Pro Leu Asp Ser Ala Arg Ser Arg Leu Ser Ser Asn Leu
   850 855 860
<210> 6
   <211> 3102
   <212> DNA
   <213> Murinae gen. sp.
<400> 6
   ccgctccgct ccgcactgcc cggcgccgcc tcgccatgga tgcgcgcggg ggcggcgggc 60
   ggccgggcga tagtccgggc acgacccctg cgccggggcc gccgccaccg ccgccgccgc 120
   ccgcgccccc tcagcctcag ccaccacccg cgccaccccc gaaccccacg accccctcgc 180
   acccggagtc ggcggacgag cccggcccgc gcgcccggct ctgcagccgc gacagcgcct 240
   gcacccctgg cgcggccaag ggcggcgcga atggcgagtg cgggcgcggg gagccgcagt 300
   gcagccccga gggccccgcg cgcggcccca aggtttcgtt ctcatgccgc ggggcggcct 360
   ccgggccctc ggcggccgag gaggcgggca gcgaggaggc gggcccggcg ggtgagccgc 420
   gcggcagcca ggctagcttc ctgcagcgcc aattcggggc gcttctgcag cccggcgtca 480
   acaagttctc cctgcggatg ttcggcagcc agaaggccgt ggagcgcgag caggaacgcg 540
   tgaagtcggc gggggcctgg atcatccacc cctacagcga cttcaggttc tactgggact 600
   tcaccatgct gttgttcatg gtgggaaatc tcattatcat tcccgtgggc atcactttct 660
   tcaaggacga gaccaccgcg ccctggatcg tcttcaacgt ggtctcggac actttcttcc 720
   tcatggactt ggtgttgaac ttccgcaccg gcattgttat tgaggacaac acggagatca 780
   tcctggaccc cgagaagata aagaagaagt acttgcgtac gtggttcgtg gtggacttcg 840
   tgtcatccat cccggtggac tacatcttcc tcatagtgga gaagggaatc gactccgagg 900
   tctacaagac agcgcgtgct ctgcgcatcg tgcgcttcac caagatcctc agtctgctgc 960
   ggctgctgcg gctatcacgg ctcatccgat atatccacca gtgggaagag attttccaca 1020
   tgacctacga cctggcaagt gcagtgatgc gcatctgtaa cctgatcagc atgatgctac 1080
   tgctctgcca ctgggacggt tgcctgcagt tcctggtgcc catgctgcaa gacttcccca 1140
   gcgactgctg ggtgtccatc aacaacatgg tgaaccactc gtggagcgag ctctactcgt 1200
   tcgcgctctt caaggccatg agccacatgc tgtgcatcgg ctacgggcgg caggcgcccg 1260
   agagcatgac agacatctgg ctgaccatgc tcagcatgat cgtaggcgcc acctgctatg 1320
   ccatgttcat tgggcacgcc actgcgctca tccagtccct ggattcgtca cggcgccaat 1380
   accaggagaa gtacaagcaa gtagagcaat acatgtcctt ccacaaactg cccgctgact 1440
   tccgccagaa gatccacgat tactatgaac accggtacca agggaagatg tttgatgagg 1500
   acagcatcct tggggaactc aacgggccac tgcgtgagga gattgtgaac ttcaactgcc 1560
   ggaagctggt ggcttccatg ccgctgtttg ccaatgcaga ccccaacttc gtcacagcca 1620
   tgctgacaaa gctcaaattt gaggtcttcc agcctggaga ttacatcatc cgagagggga 1680
   ccatcgggaa gaagatgtac ttcatccagc atggggtggt gagcgtgctc accaagggca 1740
   acaaggagat gaagctgtcg gatggctcct atttcgggga gatctgcttg ctcacgaggg 1800
   gccggcgtac ggccagcgtg cgagctgaca cctactgtcg cctctactca ctgagtgtgg 1860
   acaatttcaa cgaggtgctg gaggaatacc ccatgatgcg gcgtgccttt gagactgtgg 1920
   ctattgaccg gctagatcgc ataggcaaga agaactccat cttgctgcac aaggttcagc 1980
   atgatctcag ctcaggtgtg ttcaacaacc aggagaatgc catcatccag gagattgtca 2040
   aatatgaccg tgagatggtg cagcaggcag agcttggcca gcgtgtgggg ctcttcccac 2100
   caccgccacc accgcaggtc acatcggcca ttgccaccct acagcaggct gtggccatga 2160
   gcttctgccc gcaggtggcc cgcccgctcg tggggcccct ggcgctaggc tccccacgcc 2220
   tagtgcgccg cgcgccccca gggcctctgc ctcctgcagc ctcgccaggg ccacccgcag 2280
   caagcccccc ggctgcaccc tcgagccctc gggcaccgcg gacctcaccc tacggtgtgc 2340
   ctggctctcc ggcaacgcgc gtggggcccg cattgcccgc acgtcgcctg agccgcgcct 2400
   cgcgcccact gtccgcctcg cagccctcgc tgccccatgg cgtgcccgcg cccagccccg 2460
   cggcctctgc gcgcccggcc agcagctcca cgccgcgcct gggacccgca cccaccgccc 2520
   ggaccgccgc gcccagtccg gaccgcaggg actcagcctc gccgggcgct gccagtggcc 2580
   tcgacccact ggactctgcg cgctcgcgcc tctcttccaa cttgtgaccc ttgagcgccg 2640
   ccccgcgggc cgggcggggc cgtcatccac accaaagcca tgcctcgcgc cgcccgcccg 2700
   tgcccgtgca gaagccatag agggacgtag gtagcttagg aggcgggcgg ccctgcgccc 2760
   ggctgtcccc ccatcgccct gcgcccaccc ccatcgcccc tgccccagcg gcggccgcac 2820
   gggagaggga ggggtgcgat cacctcggtg cctcagcccc aacctgggac agggacaggg 2880
   cggccctggc cgaggacctg gctgtgcccc gcatgtgcgg tggcctccga ggaagaatat 2940
   ggatcaagtg caatacacgg ccaagccggc gtgggggtga ggctgggtcc ccggccgtcg 3000
   ccatgaatgt actgacgagc cgaggcagca gtggccccca cgccccatta acccacaacc 3060
   ccattccgcg caataaacga cagcattggc aaaaaaaaaa aa 3102
<210> 7
   <211> 17
   <212> DNA
   <213> Künstliche Sequenz
<220>
   <223> Beschreibung der künstlichen Sequenz:Primer
<220>
   <221> misc_feature
   <222> (1) .. (17)
<400> 7
   gccaatacca ggagaag 17
<210> 8
   <211> 19
   <212> DNA
   <213> Künstliche Sequenz
<220>
   <223> Beschreibung der künstlichen Sequenz:Primer
<220>
   <221> misc_feature
   <222> (1) .. (19)
   <400> 8
   tgagtagagg cgacagtag 19
<210> 9
   <211> 24
   <212> DNA
   <213> Künstliche Sequenz
<220>
   <223> Beschreibung der künstlichen Sequenz:Primer
<220>
   <221> misc_feature
   <222> (1)..(24)
<400> 9
   agtggcctcg acccactgga ctct 24
<210> 10
   <211> 25
   <212> DNA
   <213> Künstliche Sequenz
<220>
   <223> Beschreibung der künstlichen Sequenz:Primer
<220>
   <221> misc_feature
   <222> (1)..(25)
<400> 10
   ccgcctccta agctacctac gtccc 25